(19) Europäisches Patentamt
European Patent Office
Office européen des brevets

(11) EP 1 722 861 B1

(12) **FASCICULE DE BREVET EUROPEEN**

(45) Date de publication et mention de la délivrance du brevet:
**02.09.2009 Bulletin 2009/36**

(21) Numéro de dépôt: **04817582.2**

(22) Date de dépôt: **22.12.2004**

(51) Int Cl.:
***A61Q 5/12*** *(2006.01)* ***A61K 8/81*** *(2006.01)*

(86) Numéro de dépôt international:
**PCT/FR2004/003355**

(87) Numéro de publication internationale:
**WO 2005/063176 (14.07.2005 Gazette 2005/28)**

(54) **COMPOSITION COSMETIQUE COMPRENANT UN COPOLYMERE AMPHOLYTE**
KOSMETISCHE ZUSAMMENSETZUNG MIT EINEM AMPHOLYT-COPOLYMER
COSMETIC COMPOSITION COMPRISING AN AMPHOLYTE COPOLYMER

(84) Etats contractants désignés:
**AT BE BG CH CY CZ DE DK EE ES FI FR GB GR HU IE IS IT LI LT LU MC NL PL PT RO SE SI SK TR**

(30) Priorité: **23.12.2003 FR 0315281**

(43) Date de publication de la demande:
**22.11.2006 Bulletin 2006/47**

(73) Titulaire: **RHODIA CHIMIE**
**92512 Boulogne Billancourt Cedex (FR)**

(72) Inventeurs:
- **SEIGNEURIN, Aline**
**F-78150 LE CHESNAY (FR)**
- **FOUCAULT, Carole**
**F-94220 CHARENTON LE PONT (FR)**

(74) Mandataire: **Boittiaux, Vincent**
**Rhodia Services,**
**DPI,**
**40, rue de la Haie-Coq**
**93306 Aubervilliers Cedex (FR)**

(56) Documents cités:
**WO-A-01/10213**
**US-A1- 2002 052 304**
**US-B1- 6 569 261**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 722 861 B1

## Description

La présente invention concerne l'utilisation dans de nouvelles compositions cosmétiques, plus particulièrement pour le traitement de la peau ou des cheveux, d'un copolymère artificiel ampholyte.

On cherche, dans les compositions cosmétiques destinées au traitement de la peau ou des cheveux, destinées à être appliquées et rincées, comme les shampoings ou les gel-douche, à optimiser certaines propriétés comme la viscosité, la transparence, le dépôt de matière (effet conditionneur), et/ou, plus généralement, à optimiser des effets cosmétiques tels la douceur, la souplesse, le démêlage, la brillance, l'aptitude au coiffage sur cheveux secs ou mouillés. Bien entendu, outre les effets apportés par les ingrédients, on cherche également des formulations faciles à préparer, faciles mettre en oeuvre, et suffisamment stables.

Il a ainsi été proposé d'utiliser dans des formulations destinées à être rincées des dérivés de polymères naturels, comme les dérivés cationiques du guar ou de la cellulose. Il a ainsi été proposé des compositions comprenant des associations de dérivés cationiques de guar et de polyorganosiloxanes, ou des associations de dérivés cationiques de cellulose et de polyorganosiloxane. Ces systèmes, comme tous les systèmes, relèvent d'un compromis entre la quantité de polymère utilisée (que l'on souhaite faible), la transparence (que l'on souhaite élevée, mais qui diminue quand on augmente la quantité du polymère), et les propriété conditionnantes liées à un dépôt du polyorganosiloxane (que l'on souhaite élevé, et qui peut augmenter quand on augmente la quantité de polymère). Il existe donc un besoin pour de nouvelles compositions pour lesquelles ce compromis est modifié.

Il a également été proposé d'utiliser des copolymères synthétiques comprenant des unités cationiques (ou potentiellement cationiques), des unités anioniques, et éventuellement des unités neutres. Ainsi, il a été proposé d'utiliser dans certaines formulations:

- des copolymères dérivant d'acide acrylique (AA, 2 à 40% en poids) et de chlorure de chlorure de diméthyldiallylammonium (DADMAC, 60 à 90% en poids), par exemple dans les documents EP 269243 et EP 266111, EP 521748, un polymère commercial étant le Merquat 280 commercialisé par Calgon
- des copolymères dérivant d'acide acrylique (AA, 35 % en nombre), de chlorure de chlorure de diméthyldiallylammonium (DADMAC, 30% en nombre), et d'acrylamide (AM, 35% en nombre), par exemple dans les documents EP 522755, EP 522756 et EP 1115370, un polymère commercial étant le Merquat plus 3330 commercialisé par Calgon,
- des copolymères dérivant d'acide acrylique (AA), de chlorure de methacrylamidopropyltriméthylammonium (MAPTAC) et d'acrylate d'alkyl, par exemple décrits dans les documents EP 522756 et EP 1115370, un polymère commercial étant le Merquat 2001 commercialisé par Calgon. (AA/MAPTAC/Acrylate de méthyl).

Le document brevet US-B1-6 569 261 (RHODIA) décrit des copolymères DIQUAT/acrylique ou terpolymères DIQUAT/acrylique/acrylamide (polymères 1-5). Aux ex. 7-9, des compositions sont réalisées comprenant 0.05-1% du terpolymère 1. La composition comprend également 0.3% d'hydroxyde d'ammonium. Aux ex. 10-11, d'autres compositions sont réalisées, comprenant 0.5-1% du copolymère 2. Leur pH est rendu basique par la soude (pH =10.4).

Ces compositions sont indiquées pour le nettoyage et destinées au traitement de surfaces dures industrielles ou domestiques.

On cherche toujours à proposer de nouvelles compositions cosmétiques, en particulier destinées à être rincées, présentant des qualités améliorées en matière de stabilité et/ou de simplification des formulations et/ou de transparence et/ou de qualités cosmétiques (mentionnées ci-dessus) et/ou de dépôt de matière (dépôt d'un polymère portant des charges cationiques ou dépôt d'autres matières comme des huiles minérale, végétales ou synthétiques, par exemples de huiles silicones, ou "polyorganosiloxanes").

A cet effet, l'invention propose l'utilisation dans une composition cosmétique comprenant un vecteur cosmétiquement acceptable, de préférence de l'eau, éventuellement un tensioactif et un copolymère ampholyte comprenant des unités (c) cationiques et des unités (a) anioniques ou susceptibles d'être anioniques, d'un polymère qui comprend:

- 0,1 à 50% en nombre d'unités (c), dérivant de la polymérisation d'au moins un composé monomère (c) de formule générale I :

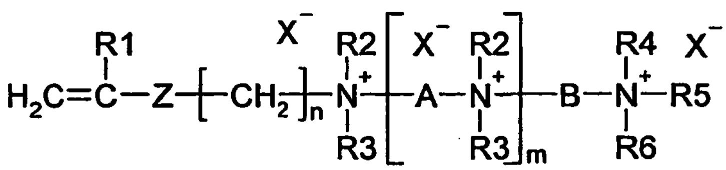

dans laquelle:

- $R_1$ est un atome d'hydrogène, un groupe méthyle ou éthyle ;
- $R_2$, $R_3$, $R_4$, $R_5$, $R_6$, identiques ou différents, sont des groupes alkyle, hydroxyalkyle ou aminoalkyle, linéaires ou ramifiés en $C_1$-$C_6$, de préférence en $C_1$-$C_4$.
- m est un nombre entier de 0 à 10, de préférence de 0 à 2 ;
- n est un nombre entier de 1 à 6, de préférence 2 à 4 ;
- Z représente un groupe -C(O)O-, -C(O)NH- ou un atome d'oxygène ;
- A représente un groupe $(CH_2)_p$, p étant un nombre entier de 1 à 6, de préférence de 2 à 4;
- B représente une chaîne polyméthylène linéaire ou ramifiée en $C_2$-$C_{12}$, avantageusement $C_3$-$C_6$, éventuellement interrompue par un ou plusieurs hétéroatomes ou hétérogroupes, notamment O ou NH, et éventuellement substituée par un ou plusieurs groupes hydroxyles ou amino, de préférence hydroxyles ;
- $X^-$, identiques ou différents, représentent des contre-ions ;

- des unités (a) dérivant de la polymérisation d'au moins un monomère (a) hydrophile portant une fonction à caractère acide copolymérisable avec (a), anionique ou susceptible d'être anionique,
- éventuellement des unités (n) dérivant d'au moins un monomère (n) à insaturation éthylénique, de charge neutre, copolymérisable avec (c) et (a), de préférence un composé monomère hydrophile à insaturation éthylénique de charge neutre portant un ou plusieurs groupes hydrophiles, copolymérisable avec (c) et (a),
- la quantité d'unités (a) et éventuellement (n) étant de 50 à 99,9 % en nombre,
- le pH de la composition est supérieur ou égal à 5,5, de préférence compris entre 5,5 et 7,5,
- la proportion en poids de tensioactif dans la composition est comprise entre 0 et 30%, de préférence entre 5 et 30% en poids, le tensioactif comprenant un tensioactif anionique et éventuellement un tensioactif amphotère,
- la proportion en poids du copolymère dans la composition est comprise entre 0,01 et 5%, de préférence entre 0,05 et 1,5 %, de préférence de 0,1 à 0,3.

Les compositions sont de préférence des compositions destinées à être rincées. Il peut s'agir par exemple d'un shampoing, d'un gel-douche ou d'un après shampoing. Il peut néanmoins s'agir d'une composition de soin capillaire qui n'est pas destiné à être rincé, par exemple un après shampoing destiné à ne pas être rincé, un lait démêlant, une eau démêlante, une eau de lissage, un revêtement de cuticule, un produit de soin capillaire coiffant et/ou recoiffant, un produit de protection solaire, une crème de soin, un démaquillant, un maquillage, des lingettes démaquillantes ou hydratantes, des mousses à raser, des mousses coiffantes ou fixantes.

Effets

L'utilisation du polymère dans la composition selon l'invention peut procurer en particulier une augmentation de la viscosité. Le polymère se dépose sur les cheveux et/ou la peau, et procure un effet recherché de conditionneur. Les compositions, lorsqu'elles comprennent un agent conditionneur, par exemple une silicone (appelée de manière équivalente un polyorganosiloxane), favorisent le dépôt dudit agent. Le polymère aide ainsi au dépôt d'agents conditionneurs, plus particulièrement de silicones (ou polyorganosiloxanes). En outre, les compositions comprenant un polyorganosiloxane et le polymère présentent d'excellentes propriétés conditionnantes, pour les cheveux ou la peau, ainsi que des propriétés sensorielles ou cosmétiques intéressantes, qui peuvent être recherchées par les consommateurs. Ainsi elles peuvent procurer un profil intéressant de douceur, de souplesse, de volume, de démêlage, d'aptitude au coiffage sur cheveux mouillé et/ou d'aptitude au coiffage sur cheveux sec. Ces effets peuvent rendre plus simples les formulations et/ou moins coûteuses. Les compositions présentent en outre des propriété de moussage satisfaisantes, notamment en eau dure.

En particulier le copolymère selon l'invention présente une grande transparence. Il peut être utilisé à titre d'agent transparent. Il permet notamment de réaliser des compostions de transparence élevée, avec une capacité de dépôt (par exemple pour conditionnement) élevée, et/ou un capacité d'aide au dépôt de polyorganosiloxanes (par exemple pour conditionnement), en particulier lors d'un rinçage. L'association de faibles quantités de polymère, typiquement de 0,1 à 0,3 % en poids de la composition, et même d'environ 0,1%, avec de polyorganosiloxanes procure des compositions de haute transparence, avec un important niveau de dépôt du polyorganosiloxane, et donc avec un profile de conditionnement et/ou un profile sensoriel ou cosmétique intéressant.

Polymère

Le copolymère comprend:

- 0.1 à 50% en nombre d'unités (c) dérivant de la polymérisation d'au moins un composé monomère (c) de formule générale I :

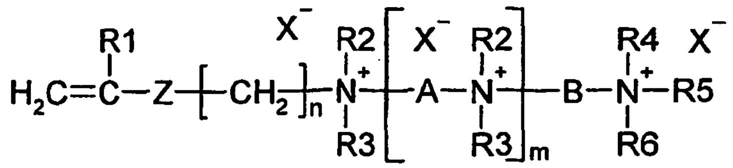

dans laquelle:

- $R_1$ est un atome d'hydrogène, un groupe méthyle ou éthyle ;
- $R_2$, $R_3$, $R_4$, $R_5$, $R_6$, identiques ou différents, sont des groupes alkyle, hydroxyalkyle ou aminoalkyle, linéaires ou ramifiés en $C_1$-$C_6$, de préférence en $C_1$-$C_4$,
- m est un nombre entier de 0 à 10, de préférence de 0 à 2 ;
- n est un nombre entier de 1 à 6, de préférence 2 à 4 ;
- Z représente un groupe -C(O)O-, -C(O)NH- ou un atome d'oxygène ;
- A représente un groupe $(CH_2)_p$, p étant un nombre entier de 1 à 6, de préférence de 2 à 4;
- B représente une chaîne polyméthylène linéaire ou ramifiée en $C_2$-$C_{12}$, avantageusement $C_3$-$C_6$, éventuellement interrompue par un ou plusieurs hétéroatomes ou hétérogroupes, notamment O ou NH, et éventuellement substituée par un ou plusieurs groupes hydroxyles ou amino, de préférence hydroxyles ;
- $X^-$, identiques ou différents, représentent des contre-ions ;

- des unités (a) dérivant de la polymérisation d'au moins un monomère (a) hydrophile portant une fonction à caractère acide copolymérisable avec (a), anionique ou susceptible d'être anionique,
- éventuellement des unités (n) dérivant d'au moins un monomère (n) à insaturation éthylénique, de charge neutre, copolymérisable avec (c) et (a), de préférence un composé monomère hydrophile à insaturation éthylénique de charge neutre portant un ou plusieurs groupes hydrophiles, copolymérisable avec (c) et (a),
- la quantité d'unités (a) et éventuellement (n) étant de 50 à 99,9 % en nombre.

L'ion $X^-$ est avantageusement choisi parmi halogènure, par exemple chlorure, sulfate, methyl sulfate, hydrosulfate, phosphate, citrate, forméate et acétate.

Le monomère (c) peut être préparé par exemple selon les schémas réactionnels suivants:

*- schéma réactionnel n° 1:*
(lorsque m est égal à 0)

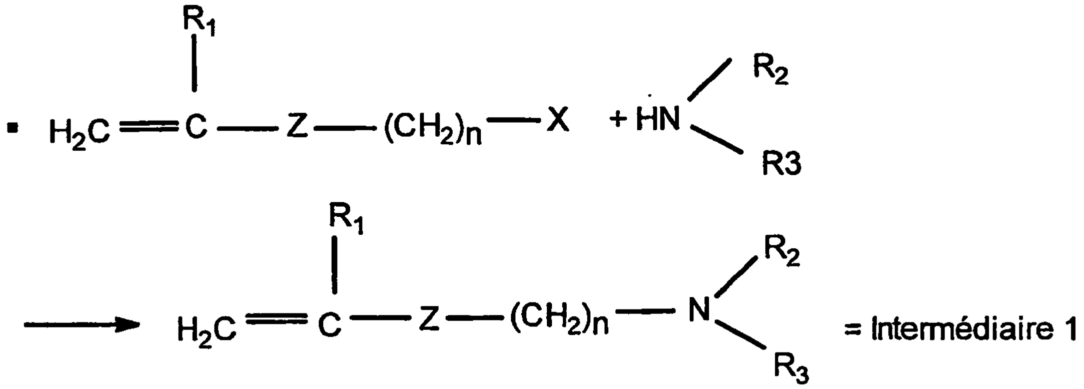

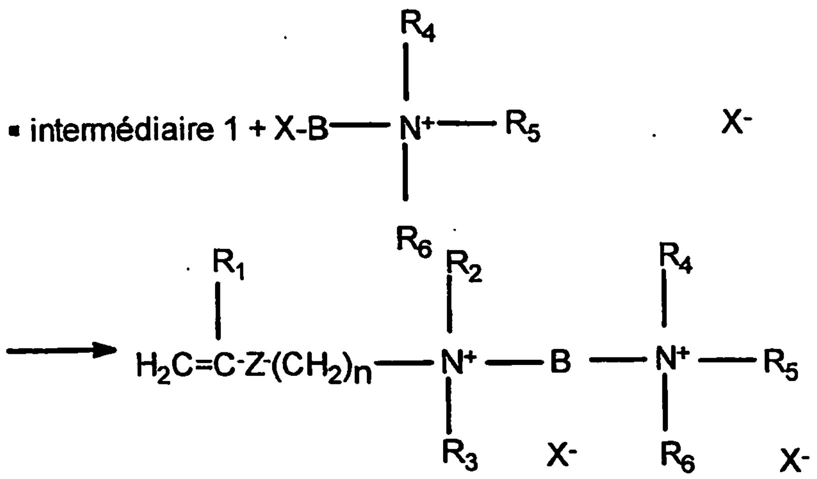


*schéma réactionnel n° 2 :*
(lorsque m est égal à 1)

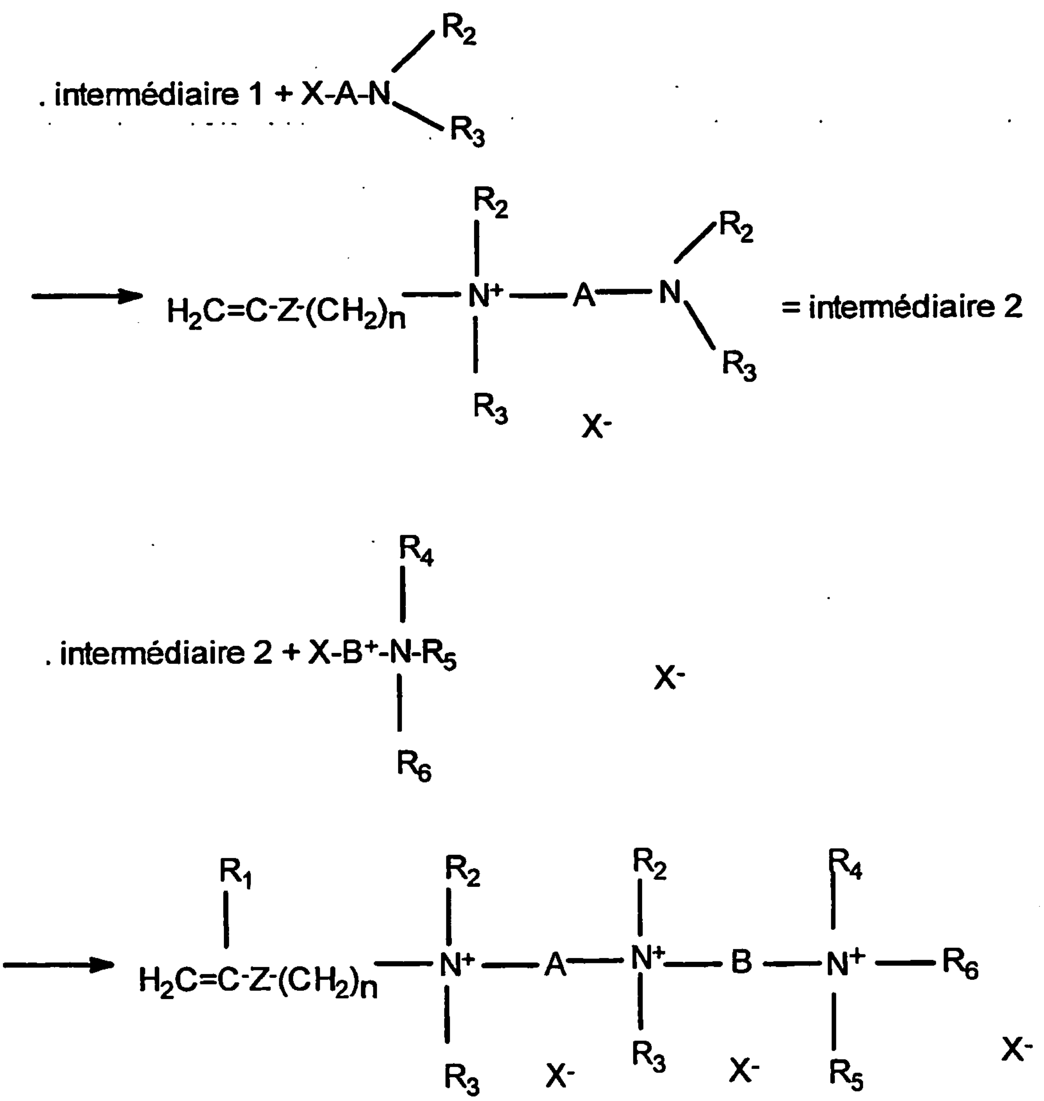

*schéma réactionnel n° 3 :*
(lorsque m est compris entre 2 et 10)

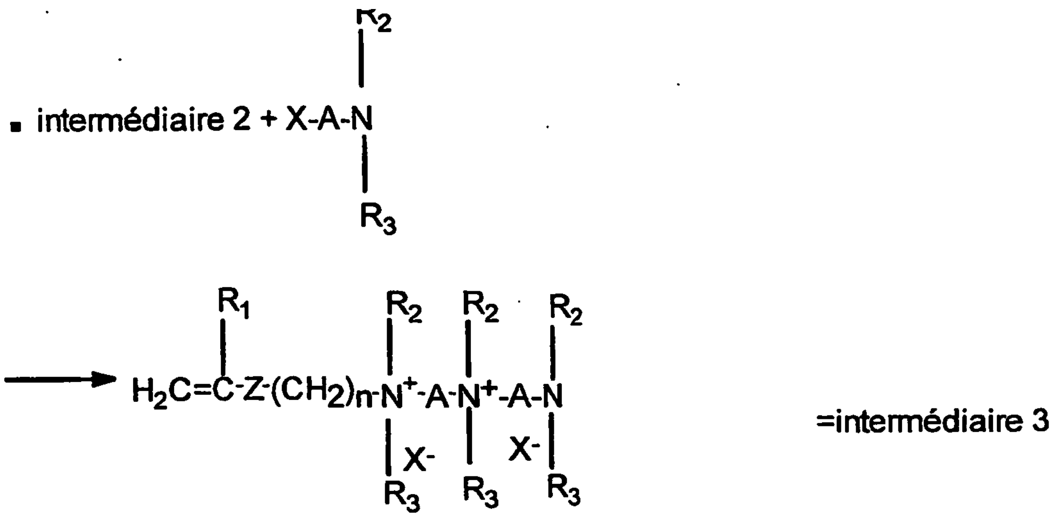


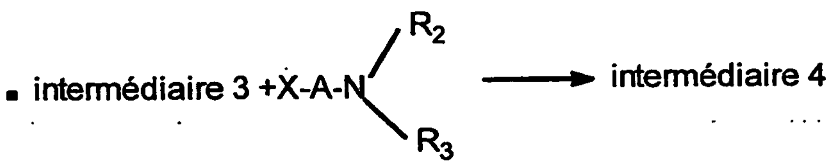


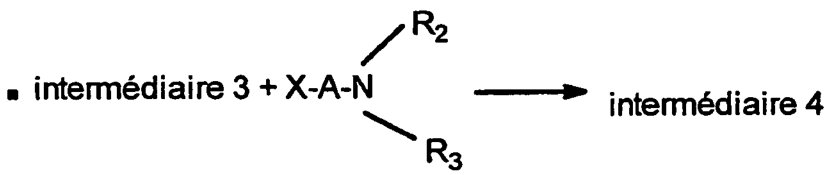


• ..... etc...... → intermédiaire m+1

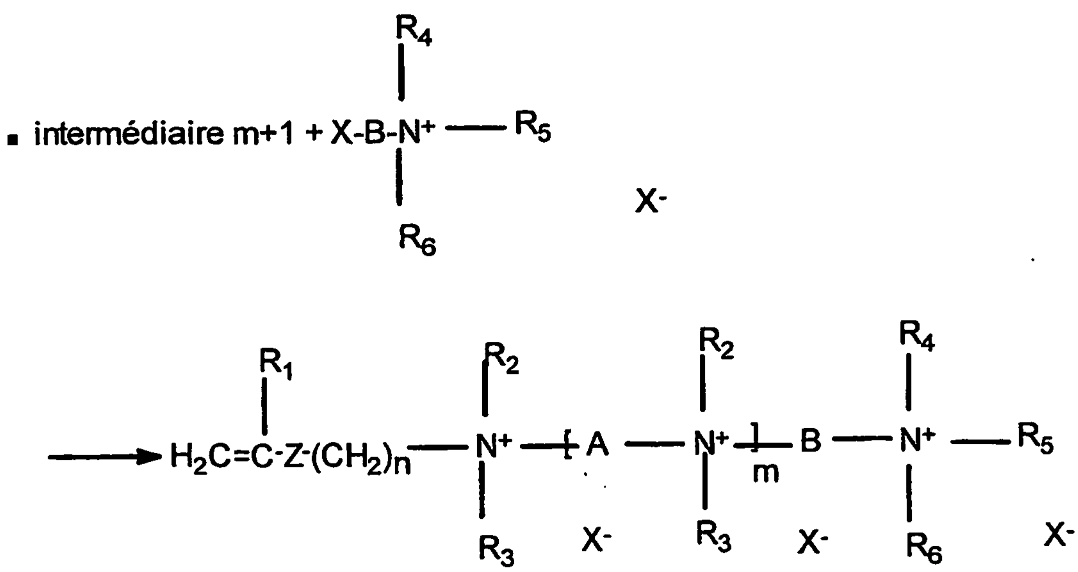

Le copolymère selon l'invention présente avantageusement une masse moléculaire d'au moins 1 000, avantageusement d'au moins 10 000 ; elle peut aller jusqu'à 20 000 000, avantageusement jusqu'à 10 000 000. Elle est de préférence comprise entre 500 000 et 5 000 000.

Sauf indications contraires, lorsqu'on parlera de masse moléculaire, il s'agira de la masse moléculaire en poids, exprimée en g/mol. Celle-ci peut être déterminée par chromatographie de perméation de gel aqueux (GPC) ou mesure de la viscosité intrinsèque dans une solution 1 N de $NaNO_3$ at 30°C.

Le copolymère est de préférence statistique.

De façon préférentielle, dans la formule générale (I) du monomère (c),

- Z représente C(O)O, C(O)NH ou O, tout préférentiellement C(O)NH ;
- n est égal à 2 ou 3, tout particulièrement 3
- m va de 0 à 2, de préférence est égal à 0 ou 1, tout particuliètrement à 0 ;
- B représente:

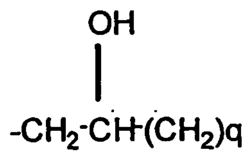

  - avec q de 1 à 4, de préférence égal à 1 ;
  - $R_1$ à $R_6$ identiques ou différents représentent un groupe méthyle ou éthyle.

Le monomère (c) préféré est le DIQUAT de formule suivante :

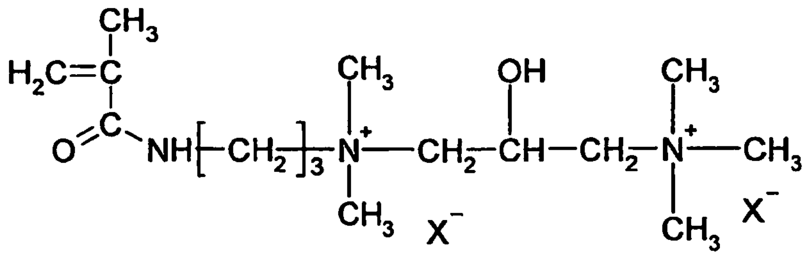

$X^-$ représentant l'ion chlorure ou méthyl sulfate.
D'autres monomères (c) particulièrement intéressants sont :

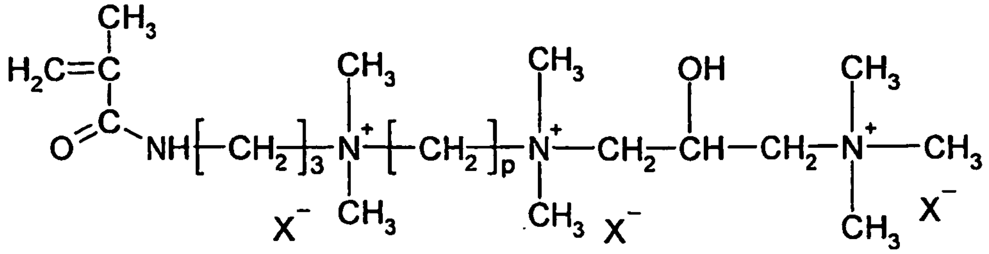

où p= 2 à 4

Les anions $X^-$ sont notamment un anion d'halogène, de préférence un anion de chlore, sulfonate, sulfate, méthyl sulfate, hydrogénosulfate, phosphate, phosphonate, citrate, formiate et acétate.

Les monomères (a) sont avantageusement des acides carboxyliques, sulfoniques, sulfuriques, phosphoniques ou phosphoriques en $C_3$-$C_8$ à insaturation monoéthylénique, leurs anhydrides et leurs sels hydrosolubles.

Parmi les monomères (a) préférés, on peut citer l'acide acrylique, l'acide méthacrylique, l'acide $\alpha$-éthacrylique, l'acide $\beta$,$\beta$-diméthylacrylique, l'acide méthylènemalonique, l'acide vinylacétique, l'acide allylacétique, l'acide éthylidineacétique, l'acide propylidineacétique, l'acide crotonique, l'acide maléique, l'acide fumarique, l'acide itaconique, l'acide

citraconique, l'acide mésaconique, la N-méthacroyl-alanine, la N-acryloyl-hydroxy-glycine, l'acrylate de sulfopropyle, l'acrylate de sulfoéthyle, le méthacrylate de sulfoéthyle, le méthacrylate de sulfoéthyle, l'acide styrène sulfonique, l'acide vinyl sulfonique, l'acide vinylphosphonique, l'acrylate de phosphoéthyle, l'acrylate de phophonoéthyle, l'acrylate de phosphopropyle, l'acrylate de phophonopropyle, le méthacrylate de phosphoethyle, le méthacrylate de phophonoethyle, le méthacrylate de phosphopropyle, le méthacrylate de phophonopropyle, et les sels de métal alcalin et d'ammonium de ceux-ci.

Parmi les monomères (n), on peut citer l'acrylamide, l'alcool vinylique, les esters d'alkyle en $C_1$-$C_4$ de l'acide acrylique et de l'acide méthacrylique, les esters d'hydroxyalkyle en $C_1$-$C_4$ de l'acide acrylique et de l'acide méthacrylique, notamment l'acrylate et le méthacrylate d'éthylène glycol et de propylène glycol, les esters polyalkoxylés de l'acide acrylique et de l'acide méthacrylique, notamment les esters de polyéthylène glycol et de polypropylène glycol, les esters de l'acide acrylique ou de l'acide méthacrylique et de polyéthylène glycol ou polypropylène glycol mono $C_1$-$C_{25}$ alkyl ethers, l'acétate de vinyle, la vinylpyrrolidone, le methylvinyléther.

Le polymère comprend de 0,1 à 50% en nombre d'unités (c), et de 50 à 99,1 % en nombre d'unités (a) et éventuellement (n). De préférence, le polymère comprend de 10 à 40% d'unités (c) et de 60 à 90% d'unités (a) et éventuellement (n). Par ailleurs, le polymère ne comprend avantageusement pas d'unités (n). Si le copolymère comprend des unités (n), le rapport molaire entre les unités (a) et le unités (n) est de préférence supérieur à 1, par exemple compris entre 1 et 4.

De préférence, le polymère est tel que

- les unités (c ) dérivent de monomère (c ) de formule suivante:

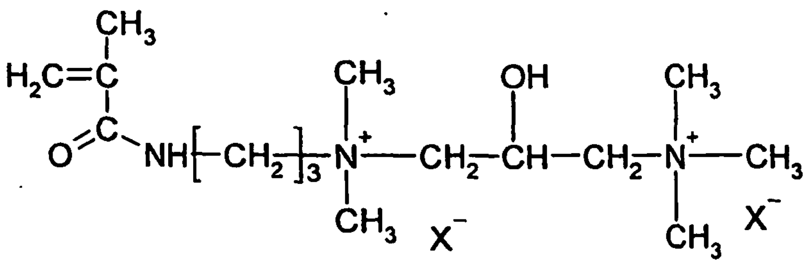

X$^-$ représentant l'ion chlorure ou méthyl sulfate,
- les unités (a) dérivent d'acide acrylique,
- le polymère ne comprend pas d'unités (n),
- le rapport en nombre entre les unités (a) et les unités (c) est de 50/50 à 90/10.

On précise que les copolymères peuvent avoir une charge moyenne négative ou nulle, au pH de la composition ou au pH d'utilisation de la composition. Cette charge moyenne est définie par l'équation suivante:

$$Q = \frac{[c]X_c - [a]X_a}{[c]X_c + [a]X_a}$$

où:

- [c] est la concentration molaire en unités (c),
- [a] est la concentration molaire en unités (a),
- $X_C$ représente le taux de neutralisation éventuelle des unités (a)$A_C$ (dans le cas ou les unités (a) sont potentiellement cationiques); $X_C = [BH^+]/([B] + [BH^+])$,
- $X_A$ représente le taux de neutralisation éventuelle des unités (c) (dans le cas ou les unités (c) sont potentiellement anioniques); $X_A = [A^-]/([AH] + [A^-])$.

Les copolymères de l'invention peuvent être obtenus selon les techniques connues de préparation des copolymères, notamment par polymérisation par voie radicalaire des monomères de départ éthyléniquement insaturés qui sont des composés connus ou pouvant être facilement obtenus par l'homme du métier en mettant en oeuvre des

procédés de synthèse classique de chimie organique.

On pourra notamment se référer aux procédés décrits dans US 4387017 et EP156646.

La polymérisation radicalaire est de préférence conduite dans un environnement exempt d'oxygène, par exemple en présence d'un gaz inerte (hélium, argon, etc) ou d'azote. La réaction est effectuée dans un solvant inerte, de préférence le méthanol ou l'éthanol, et de façon plus préférée dans l'eau.

La polymérisation est initiée par addition d'un initiateur de polymérisation. Les initiateurs utilisés sont les initiateurs de radicaux libres habituellement utilisés dans la technique. Des exemples comprennent les peresters organiques (t-butylperoxypivalate, t-amylperoxypivalate, t-butylperoxy-ac-éthylhexanoate, etc) ; des composés organiques de type azo, par exemple le chlorhydrate d'azo-bis-amidino-propane, l'azo-bis-isobutyronitrile, l'azo-bis-2,4-diméthylvaléronitrile, etc) ; les peroxydes inorganiques et organiques, par exemple le peroxyde d'hydrogène, le peroxyde de benzyle et le peroxyde de butyle, etc ; des systèmes d'initiateurs redox, par exemple ceux comprenant des agents oxydants, tels que les persulfates (notamment les persulfates d'ammonium ou de métaux alcalins, etc) ; les chlorates et les bromates (y compris les chlorates et/ou les bromates inorganiques ou organiques) ; les agents réducteurs tels que les sulfites et bisulfites (y compris les sulfites ou bisulfites inorganiques et/ou organiques) ; l'acide oxalique et l'acide ascorbique ainsi que les mélanges de deux ou plusieurs de ces composés.

Les initiateurs préférés sont des initiateurs solubles dans l'eau. On préfère en particulier le persulfate de sodium et le chlorhydate d'azo-bis-amidinopropane.

En variante, la polymérisation peut être initiée par irradiation à l'aide de lumière ultra-violette. La quantité d'initiateurs utilisés est en général, une quantité suffisante pourra réaliser l'initiation de la polymérisation. De préférence, les initiateurs sont présents en une quantité allant de 0,001 à environ 10 % en poids par rapport au poids total des monomères, et de préférence sont compris en une quantité inférieure à 0,5 % en poids par rapport au poids total des monomères, une quantité préférée étant située dans la plage de 0,005 à 0,5 % en poids par rapport au poids total des monomères. L'initiateur est ajouté au mélange de polymérisation, soit de manière continue soit de manière discontinue.

Lorsque l'on veut obtenir des copolymères de masse moléculaire élevée, il est souhaitable de rajouter de l'initiateur frais pendant la réaction de polymérisation. L'addition graduelle ou discontinue permet également une polymérisation plus efficace et un temps de réaction moins long. La polymérisation est réalisée dans des conditions réactionnelles efficaces pour polymériser les monomères (c), les monomères (a) et éventuellement les monomères (n) en atmosphère exempte d'oxygène. De préférence, la réaction est conduite à une température allant d'environ 30° à environ 100° et de préférence entre 60° et 90°C. L'atmosphère exempte d'oxygène est maintenue pendant toute la durée de la réaction, par exemple par maintien d'une purge d'azote tout au long de la réaction.

Un copolymère particulièrement préféré est le suivant :

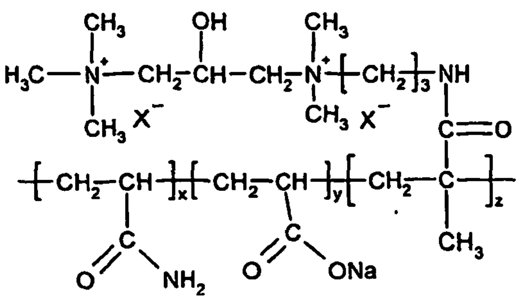

avec

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%,
- x+y+z = 100%, x, y et z représentant les % molaires de motifs dérivés respectivement d'acrylamide, acide acrylique (sel de sodium) et de DIQUAT.

D'autres polymères sont les suivants :

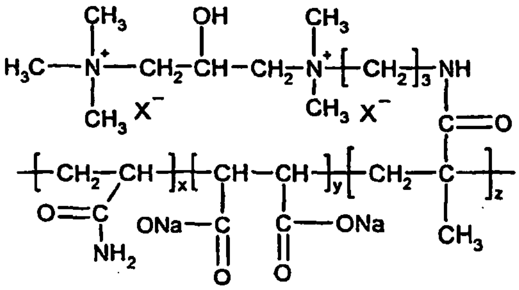

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

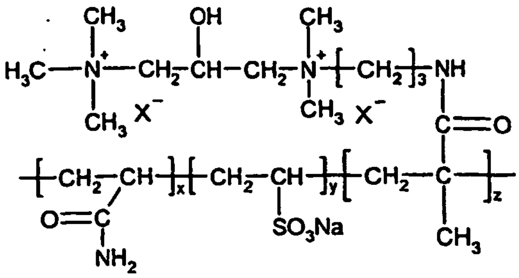

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

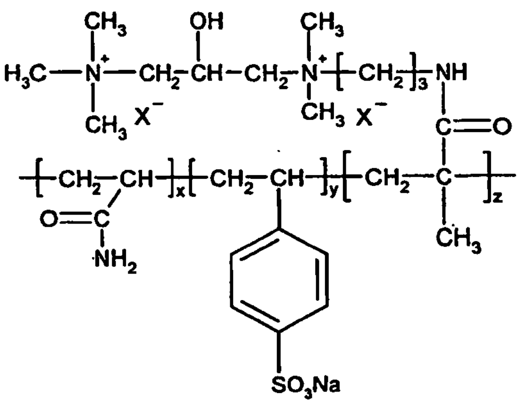

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

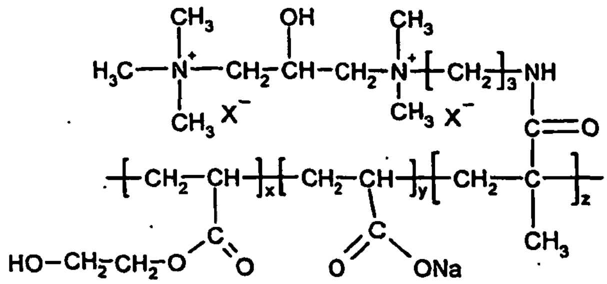

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

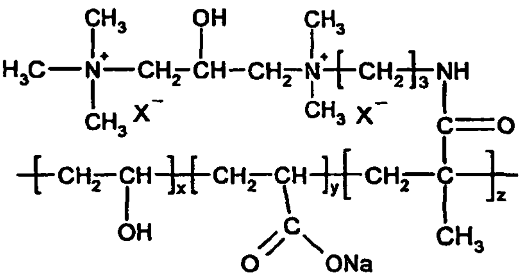

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

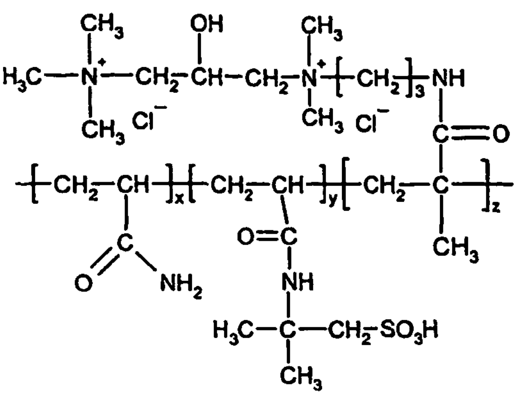

- x ayant une valeur moyenne en nombre de 0 à 50%, de préférence de 0 à 30%, de préférence égale à 0
- y ayant une valeur moyenne en nombre de 10 à 95%, de préférence de 50 à 70%,
- z ayant une valeur moyenne en nombre de 0,1 à 50%, de préférence de 10 à 50%,
- le rapport y/z étant de manière préférée de l'ordre de 4/1 à 1/2, de préférence entre 4/1 et 1/1,
- x+y est de 50 à 99,9%.

La composition comprend avantageusement de 0.1% à 5% en poids du polymère, de préférence de 0.3 à 1.5 % en poids. On précise que le polymère peut être introduit dans la composition sous forme d'une solution aqueuse plus

ou moins concentrée. Les quantités mentionnées ci-dessus sont des quantités exprimées en extrait sec.

Vecteur cosmétiquement acceptable

Tout vecteur cosmétiquement acceptable permettant de formuler le polymère ampholyte et d'obtenir la forme de composition cosmétique désirée, pour l'utilisation visée, peut être utilisé. Différents vecteurs cosmétiquement acceptables pour différents types de formulations sont connus de l'homme du métier.

A titre d'exemples de vecteurs cosmétiquement acceptables, on peut citer les vecteurs aqueux (comprenant de l'eau), les vecteurs alcooliques (comprenant un alcool, par exemple de l'éthanol, l'isopropanol, l'éthylène glycol ou les polyéthylène glycol), le propylène glycol, les vecteurs hydro-alcooliques (comprenant un mélange d'eau et d'un alcool par exemple de l'éthanol, l'isopropanol, l'éthylène glycol ou les polyéthylène glycol). Certaines huiles, volatiles ou non, peuvent également être utilisées. On cite par exemple les silicones fluides, tels que le cyclopentasiloxane, par exemple le Mirasil CM5 commercialisé par Rhodia.

L'homme du métier sait choisir les vecteurs adaptés aux types de formulations souhaités, et aux utilisations visées. Par exemple des vecteurs aqueux sont généralement utilisés pour des shampoings ou gels-douche. Un vecteur propylène glycol peut être utilisé des compositions sous forme de crèmes. Un vecteur cyclométhicone peut être utilisé pour des compositions de maquillage, par exemple pour des fonds de teint.

Composition

Tensioactifs

La composition est une composition aqueuse comprenant éventuellement des tensioactifs. Il peut s'agir d'un mélange de différents tensioactifs. Les tensioactifs compris dans la composition comprennent de préférence au moins un tensioactif anionique. Les tensioactifs peuvent également comprendre des tensioactifs amphotères (amphotères vrais ou zwitterioniques), des tensioactifs neutres (tensioactifs non ioniques) et/ou des tensioactifs cationiques. Les compositions comprenant au moins un tensioactif anionique et au moins un tensioactif amphotère sont particulièrement avantageuses, notamment pour des raisons de douceur. La teneur totale en tensioactifs dans la composition est comprise entre 0 et 30% en poids.

Pour des compositions destinées au traitement des cheveux, comme des shampoings, la teneur en tensioactif est avantageusement comprise entre 10 et 20% en poids. De telles compositions peuvent comprendre des sels, par exemple du chlorure de sodium ou d'ammonium, avantageusement en teneur inférieure à 3%en poids.

Pour des compositions destinées au traitement de la peau, comme des gel-douche, la teneur en tensioactif est avantageusement comprise entre 5 et 15% en poids. De telles compositions comprennent également de préférence au moins 2% en poids de sels, par exemple du chlorure de sodium ou d'ammonium.

Pour des après-shampoings, la teneur en tensioactifs peut être inférieure à 5% en poids.

La proportion en poids de tensioactifs anioniques par rapport à l'ensemble des tensioactifs est de préférence supérieure à 50%, de préférence supérieure à 70%.

Paramètres (pH)

Le pH de la composition est supérieur ou égal à 5,5. Il est par exemple compris entre 5,5 et 7,5, de préférence entre 6 et 6,5. Le pH dépend évidemment des composés présents dans la composition. On peut évidemment utiliser dans la composition des agents de régulation du pH, acides ou bases, par exemple de l'acide citrique, ou de l'hydroxyde de sodium, de potassium ou d'ammonium.

Natures des tensioactifs

Les tensioactifs anioniques peuvent être choisis parmi les tensioactifs suivants:

- les alkylesters sulfonates, par exemple de formule $R\text{-}CH(SO_3M)\text{-}CH_2COOR'$, ou les alkylesters sulfates, par exemple de formule $R\text{-}CH(OSO_3M)\text{-}CH_2COOR'$, où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalino-terreux, par exemple sodium, ou le cation ammonium. On peut citer tout particulièrement les méthyl ester sulfonates dont le radical R est en $C_{14}$-$C_{16}$;
- les alkylbenzènesulfonates, plus particulièrement en $C_9$-$C_{20}$, les alkylsulfonates primaires ou secondaires, notamment en $C_8$-$C_{22}$, les alkylglycérol sulfonates ;
- les alkylsulfates par exemple de formule $ROSO_3M$, où R représente un radical alkyle ou hydroxyalkyle en $C_{10}$-$C_{24}$, de préférence en $C_{12}$-$C_{20}$ ; M un cation de même définition que ci-dessus ;

- les alkyléthersulfates par exemple de formule $RO(OA)_nSO_3M$ où R représente un radical alkyle ou hydroxyalkyle en $C_{10}$-$C_{24}$, de préférence en $C_{12}$-$C_{20}$ ; OA représentant un groupement éthoxylé et/ou propoxylé ; M représentant un cation de même définition que ci-dessus, n variant généralement de 1 à 4, comme par exemple le lauryléthersulfate avec n = 2 ;
- les alkylamides sulfates, par exemple de formule $RCONHR'OSO_3M$ où R représente un radical alkyle en $C_2$-$C_{22}$, de préférence en $C_6$-$C_{20}$, R' un radical alkyle en $C_2$-$C_3$, M représentant un cation de même définition que ci-dessus, ainsi que leurs dérivés polyalcoxylés (éthoxylés et/ou propoxylés) (alkylamidoether sulfates
- les sels d'acides gras saturés ou insaturés, par exemple comme ceux en $C_8$-$C_{24}$, de préférence en $C_{14}$-$C_{20}$ et d'un cation alcalino-terreux, les N-acyl N-alkyltaurates, les alkyliséthionates, les alkylsuccinamates et alkylsulfo succinates, les monoesters ou diesters de sulfosuccinates, les N-acyl sarcosinates, les polyéthoxycarboxylates ;
- les mono et di esters phosphates, par exemple de formule suivante : $(RO)_x$-P(=O)$(OM)_x$ ou R représente un radical alkyle, alkylaryle, arylalkyle, aryle, éventuellement polyalcoxylés, x et x' étant égaux à 1 ou 2, à la condition que la somme de x et x' soit égale à 3, M représentant un cation alcalino-terreux ;

Les tensioactifs non ioniques peuvent être choisis parmi les tensioactifs suivants:

- les alcools gras alcoxylés ;
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés
- les produits résultant de la condensation de l'oxyde d'éthylène avec un composé hydrophobe résultant de la condensation de l'oxyde de propylène avec le propylène glycol, tels les Pluronic commercialisés par BASF ;
- les produits résultant de la condensation de l'oxyde d'éthylène le composé résultant de la condensation de l'oxyde de propylène avec l'éthylènediamine, tels les Tetronic commercialisés par BASF ;
- les alkylpolyglycosides comme ceux décrits dans US 4565647 ;
- les amides d'acides gras par exemple en $C_8$-$C_{20}$ ;

Les tensioactifs amphotères (amphotères vrais comprenant un groupe ioniques et un groupe potentiellement ionique de charge opposée, ou zwitterioniques comprenant simultanément deux charges opposées) peuvent être choisis parmi les tensioactifs suivants:

- les bétaïnes de manière générale, notamment carboxybétaïnes de par exemple la lauryl bétaïne (Mirataine BB de la société Rhodia) ou l'octylbétaïne; les amidoalkylbétaïnes, comme la cocamidopropyl bétaïne (CAPB) (Mirataine BDJ de la société Rhodia Chimie) ;
- les sulfo-bétaïnes ou sultaines comme la cocamidopropyl hydroxy sultaïne (Mirataine CBS de la société Rhodia) ;
- les alkylamphoacétates et alkylamphodiacétates, comme par exemple comprenant une chaîne coco, lauryle (Miranol C2M, C32, L32 notamment, de la société Rhodia) ;
- les alkylamphopropionates ou les alkylamphodipropionates, (Miranol C2M SF) ;
- les alkyl amphohydroxypropyl sultaïnes (Miranol CS).

Les tensioactifs cationiques peuvent être choisis parmi les sels d'amines grasses primaires, secondaires ou tertiaires, éventuellement polyethoxylées, les sels d'ammonium quaternaires tels que les chlorures ou les bromures de tetraalkylammonium, d'alkylamidoalkylammonium, de trialkylbenzylammonium, de trialkylhydroxyalkylammonium, ou d'alkylpyridinium, les dérivés d'imidazoline, les oxydes d'amines à caractère cationique.

A titre d'exemples de compositions utiles, on peut citer.

- Les compositions «sodium» pour shampoings comprenant typiquement 12 à 16% en poids d'alkylethersulfate de sodium (par exemple laurylethersulfate de sodium « SLES ») ou d'un mélange d'alkylethersulfate de sodium et d'alkylsulfate de sodium (par exemple laurylsulfate de sodium «SLS»), 1 à 3 % d'un tensioactif amphotère (par exemple cocoamidopropylbétaïne « CAPB »), 0.5 à 2 % d'un sel (par exemple chlorure de sodium).
- Les compositions «ammonium» pour shampoings comprenant typiquement 12 à 16% en poids d'alkylethersulfate d'ammonium (par exemple laurylethersulfate d'ammonium « ALES ») ou d'un mélange d'alkylethersulfate d'ammonium et d'alkylsulfate d'ammonium (par exemple laurylsulfate d'ammonium «ALS»), 1 à 3 % d'un tensioactif amphotère (par exemple cocoamidopropylbétaïne « CAPB »), 0 à 2 % d'un sel (par exemple chlorure d'ammonium).

- Les compositions «sodium» pour gel-douche comprenant typiquement 6 à 10% en poids d'alkylethersulfate de sodium (par exemple laurylethersulfate de sodium « SLES ») ou d'un mélange d'alkylethersulfate de sodium et d'alkylsulfate de sodium (par exemple laurylsulfate de sodium «SLS»), 1 à 3 % d'un tensioactif amphotère (par exemple cocoamidopropylbétaïne « CAPB »), 2 à 4 % d'un sel (par exemple chlorure de sodium).
- Les compositions «sodium» pour gel-douche comprenant typiquement 6 à 10% en poids d'alkylethersulfate d'ammonium (par exemple laurylethersulfate d'ammonium « ALES ») ou d'un mélange d'alkylethersulfate d'ammonium et d'alkylsulfate d'ammonium (par exemple laurylsulfate d'ammonium «ALS»), 1 à 3 % d'un tensioactif amphotère (par exemple cocoamidopropylbétaïne « CAPB »), 0 à 4 % d'un sel (par exemple chlorure d'ammonium).

Autres composés

La composition peut comprendre tout autre composé utilisé dans les compositions cosmétiques destinées à être rincées (shampoing, gel-douche, après-shampoings...), ou destinées à ne pas être rincées.

On cite par exemple les séquestrants, les adoussissants, les modificateurs de mousse, les colorants, les agents nacrant (pearlizers), les agents hydratants, les agents antipelliculaires ou antiséborrhéiques, les agents de mise en suspension, les agents de mise en émulsion, les céramides, les pseudocéramides, les éléctrolytes, les acides gras, les esters d'acides gras, les hydroxyacides, les épaississants, les parfums, les conservateurs, les filtres solaires organiques ou minéraux, les protéines, les vitamines, des polymères, des silicones "polyorganosiloxanes", des agents de stabilisation et/ou de conditionnement et/ou d'aide au conditionnement, autres que le copolymère ampholyte et que les polyorganosiléxanes, notamment des polymères. Certains de ces composés sont détaillés ci-dessous.

Agent de stabilisation et/ou de conditionnement et/ou d'aide au conditionnement

La composition cosmétique selon l'invention peut avantageusement comprendre au moins un agent de stabilisation et/ou de conditionnement (agents conditionneurs) et/ou d'aide au conditionnement. On parle aussi parfois d'agents de suspension. Par aide au conditionnement, on entend que la présence de l'agent améliore le conditionnement lié à d'autres composés, par exemple des huiles ou silicones. Les agents sont entendus comme des agents différents du polyorganosiloxane de formule (I). De tels agents sont connus de l'homme du métier. La composition selon l'invention peut comprendre plusieurs de ces agents (mélanges ou combinaisons), pour associer leurs effets et/ou créer des synergies. Par ailleurs, certains agents peuvent exercer plusieurs fonctions. C'est le cas par exemple des polysaccharides, et leurs dérivés cationiques, par exemple des dérivés cationiques de guars.

La proportion en poids de tels agents peut être typiquement de 0,1% à 10% en poids, de préférence de 0,3% à 8% en poids, pour des polysaccharides ou d'autres agents.

A titre d'exemples d'agents de stabilisation (ou agents stabilisants), particulièrement utiles pour des compositions comprenant des polyorganosiloxanes, on peut citer:

- les polyacrylates réticulés, par exemple les polymères de type CARBOPOL ou CARBOMER commercialisés par BF Goodrich ou Noveon, ACRITAMER. commercialisés par RITA ou TEGO CARBOMER commercialisés par Goldschmidt. Ces composés peuvent être typiquement présents en quantité de 0,1 à 3%, de préférence de 0,3 à 2%, en poids par rapport à la composition.
- les copolymères des acrylates/aminoacrylates/ itaconates PEG 20 alkyles $C_{10}$-$C_{30}$ commercialisés par National Starch sous le nom STRUCTURE PLUS. Ces composés peuvent être typiquement présents en quantité de 0,1 à 3%, de préférence de 0,3 à 2%, en poids par rapport à la composition.
- les solides insolubles formant un réseau dans la composition. Il peut s'agir de mono et/ou di-esters d'acides gras d'éthylène glycol, les acides gras étant de préférence en $C_{16}$-$C_{18}$. Il peut en particulier s'agir d'éthylène glycol distéarate (EGDS), par exemple commercialisé par Rhodia en concentré avec d'autres ingrédient sous le nom MIRASHEEN. Ce composé peut être typiquement présent en quantité de 3 à 10%, de préférence de 5 à 8% en poids par rapport à la composition.

On peut citer également des agents viscosants, gelifiants ou texturants comme les copolymères acryliques anioniques de type ACULYNE commercialisés par ISP ou Rohm & Haas), les polysaccharides et leurs dérivés non cationiques tels que les dérivés de la cellulose comme l'hydroxypropylcellulose, la carboxyméthylcellulose, les dérivés non-ioniques de guars comme l'hydroxypropyl guar (par exemple les Jaguar HP commercialisé par Rhodia), la caroube, la gomme de tara ou de cassia, la gomme xanthane (par exemple les Rhodicare commercialisés par Rhodia), les succinoglycanes (par exemple le Rheozan commercialisé par Rhodia), les alginates, les carraghénannes, les dérivés de la chitine ou tout autre polysaccharide à fonction texturante. Ces polysaccharides et leurs dérivés peuvent être incorporés seuls ou en combinaison synergique à d'autres polysaccharides. Ces composés peuvent être typiquement présents en quantité de 0,1 à 3%, de préférence de 0,3 à 1 %, en poids par rapport à la composition.

A titre d'exemples d'agents de stabilisation et/ou d'agents de conditionnement et/ou d'aide ou conditionnement, on peut citer:

- les polymères cationiques dérivés de polysaccharides, par exemple les dérivés cationiques de celluloses, les dérivés cationiques d'amidons, les dérivés cationiques de guars, les dérivés cationique de caroube.
- les polymères cationiques synthétiques,
- les mélanges ou combinaisons de ces agents.

Les polymères cationiques, synthétiques ou non, pouvant assurer une fonction d'agent de conditionnement, sont notamment des polymères de type polyquaternium, comme par exemple les polyquatemium-1, polyquaternium-2, polyquatemium-4, polyquatemium-5, polyquatemium-6 (également connu comme Merquat 1000 disponible auprès de Nalco), polyquatemium-7 (également connu comme Merquat 5500 disponible auprès de Nalco), polyquaternium-8, polyquatemium-9, polyquatemium10 (également connu comme Polymer JR 400, commercialisé par Amercol), polyquaternium-11, polyquaternium-12, polyquatemium-13, polyquatemium-14, polyquatemium-15, polyquatemium-16, polyquaternium-17, polyquatemium-18, polyquaternium-19, polyquatemium-20, polyquatemium-22 (également connu comme Merquat 280, 281, 298 disponibles auprès de Nalco), polyquatemium-24, polyquaternium-27, polyquatemium-28, polyquatemium-29 (également connu comme Kytamer KCO disponible auprès de Amerchol), polyquatemium-30, polyquatemium-31, polyquatemium-32, polyquatemium-33, polyquatemium-34, polyquatemium-35, polyquatemium-36, polyquatemium-37, polyquatemium-39 (également connu comme Merquat 3300, 3331 disponibles auprès de Nalco), polyquatemium-44, polyquaternium-27 (également connu comme Merquat 2001 disponible auprès de Nalco) et polyquaternium-55.

Comme mentionné ci-dessus la composition peut comprendre d'autres polymères, synthétiques ou naturels ou issus de procédé de préparation biologiques, le cas échéant fonctionnalisés, par exemple par des groupe cationiques ou neutres. Ces polymères peuvent avoir une action de stabilisation, de structuration des compositions, et/ou une action de conditionneur (dépôt à la surface de la peau ou des cheveux).

On cite à titre d'exemples les dérivés cationiques de polysaccharides, comme les dérivés de guar ou de cellulose. Des polymères cationiques fonctionnalisés par des groupes hydrophobes comme des chaînes alkyles en C1-C14, de préférence en C2-C8, présentant éventuellement un groupement hydroxyle, peuvent être utilisés. Ces groupes hydrophobes sont rattachés à la chaîne polymérique principale par l'intermédiaire de liaisons éthers.

Par ailleurs, et dans le cas des guars cationiques modifiés hydrophobes ou non, le groupement cationique est un groupement ammonium quaternaire portant trois radicaux, identiques ou non, choisis parmi l'hydrogène, un radical alkyle comprenant 1 à 22 atomes de carbone, plus particulièrement 1 à 14, de manière avantageuse 1 à 3 atomes de carbone. Le contre ion est un halogène, de préférence le chlore.

Dans le cas des celluloses cationiques modifiées hydrophobes ou non, le groupement cationique est un groupement ammonium quaternaire portant trois radicaux, identiques ou non, choisis parmi l'hydrogène, un radical alkyle comprenant 1 à 10 atomes de carbone, plus particulièrement 1 à 6, de manière avantageuse 1 à 3 atomes de carbone. Le contre ion est un halogène, de préférence le chlore. Parmi les dérivés cationiques de guar, on peut citer le guar hydroxypropyl trimonium chlorure (JAGUAR C13S, C14S, ou C17, JAGUAR Excel, JAGUAR C 2000 commercialisés par la société Rhodia Chimie) ou l'hydroxypropyl guar hydroxypropyl trimonium chlorure (JAGUAR C162 commercialisés par RHODIA).

Parmi les dérivés cationiques de cellulose, on pourra utiliser l'éther de poly(oxyéthanediyl-1,2) hydroxy-2 chlorure de triméthylammonium-3 propyl cellulose ou polyquatemium-10, comme le Polymer JR400 (dénomination INPI: PQ10) commercialisé par la société Amerchol.

Peuvent également être utilisés des dérivés non ioniques de polysaccharides, par exemple du guar hydroxypropyl.

Peuvent de même convenir, des polymères synthétiques, et plus particulièrement des homopolymères comme le polyméthacrylamidopropyl trimonium chlorure (Polycare 133 commercialisé par la société Rhodia Chimie).

Les polymères cationiques présentent plus particulièrement une masse molaire en poids d'au moins 2000 g/mol et plus préférentiellement comprise entre $2.10^4$ et $3.10^6$ g/mol, selon leur degré de polymérisation éventuelle. Les masses molaires en poids des polymères sont habituellement mesurées par exclusion de taille. Eventuellement, elles peuvent être mesurées directement par diffusion de la lumière ou à partir de la viscosité intrinsèque eh utilisant un étalonnage selon : "Viscosity-Molecular weight relationship, intrinsic chain flexibility and dynamic solution properties of guar galactomannan" de G. Robinson, S.B. Ross Murphy, E.R. Morris, Carbohydrate Research 107, p.17-32, 1982. Dans le cas des dérivés cationiques des polysaccharides, le degré d'hydroxyalkylation (substitution molaire ou MS) est de préférence compris entre 0 et 1,2. Toujours dans le cas de ces polymères, le degré de cationicité (degré de substitution ou DS) est plus particulièrement compris entre 0,01 et 0,6. C'est par exemple le cas des Jaguars C162 et C2000 commercialisés par la société Rhodia Chimie.

Polyorganosiloxanes (silicone)

La composition peut comprendre une silicone (huile silicone). On entend par silicone ou polyorganosiloxane tout composé organosiloxane, comprenant des groupes alkyle (par exemple méthyle) et/ou fonctionnalisés par des groupes différents des groupes alkyle.

Le polyorganosiloxane est avantageusement (dans les shampoings et après-shampoings en particulier) un polyorganosiloxane non volatil et non-hydrosoluble. Il présente avantageusement une viscosité comprise entre 1000 et 2000000 mPa.s, de préférence entre 5000 et 500000 mPa.s. Le polyorganosiloxane peut notamment être un polydiméthylorganosiloxanesiloxane ("PDMS", dénomination INCI: dimethicone), ou un polyorganosiloxane présentant des groupes amines (par exemple de l'Amodimethicone selon la dénomination INCI), ammonium quaternaire (par exemple les silicone quatemum 1 à 10 selon la dénomination INCI), hydroxyle (terminaux ou non), ou polyoxyalkylène, par exemple polyoxyde d'éthylène et/ou polyoxyde de propylène (en groupes terminaux, en bloc en sein d'une chaîne de PDMS, ou en greffons), ou plusieurs de ces groupes.

La quantité de polyorganosiloxane présent dans la composition peut typiquement être de 0,1 % à 5% en poids, par exemple de 0,5 % à 1,5% ou 2%.

Le polyorganosiloxane (silicones) sont de préférence présentes dans la composition sous forme d'émulsion (gouttelettes liquides de silicone dispersée dans la phase aqueuse). L'émulsion peut notamment être une émulsion dont la taille moyenne des gouttelettes est supérieure ou égale à 2 μm, et/ou dont la taille moyenne des gouttelettes est supérieure ou comprise entre 0,15 μm et 2 μm, ou dont la taille moyenne des gouttelettes est inférieure ou égale à 0,15 μm.

Les gouttelettes de l'émulsion peuvent être de taille plus ou moins importante. On peut ainsi se référer à des microémulsions, à des mini-émulsions, ou à des macro-émulsions. Dans la présente demande, le terme «émulsion» couvre notamment tous ces types d'émulsions. Sans vouloir être lié à une quelconque théorie, on précise que les microémulsions sont généralement des systèmes thermodynamiquement stables, comprenant généralement d'importantes quantités d'agents d'émulsification. Les autres émulsions sont généralement des systèmes en état non-thermodynamiquement stable, conservant pendant un certain temps, en état métastable l'énergie mécanique fournie lors de l'émulsification. Ces systèmes comprennent généralement des quantités moindres d'agents d'émulsification.

Les émulsions peuvent être obtenues par mélange du vecteur, de préférence aqueux, du polyorganosiloxane, et en général d'un agent d'émulsification, puis émulsification. On peut parler d'émulsification in situ.

Les compositions sous forme d'émulsion peuvent également être obtenues par mélange du vecteur, de préférence aqueux, avec une émulsion préalablement préparée de gouttelettes comprenant le polyorganosiloxane dans une phase externe, de préférence miscible avec le vecteur cosmétiquement acceptable, de préférence de même nature que ledit vecteur, de préférence un vecteur aqueux. On peut préférer ce mode de réalisation car il est simple à mettre en oeuvre. En outre ce mode de réalisation est particulièrement adapté pour la mise en oeuvre de compositions cosmétiques dans lesquelles le polyorganosiloxane est sous forme de microémulsion. On peut parler d'émulsification préalable.

Selon un mode particulier de réalisation, l'émulsion est une microémulsion, dont la taille des gouttelettes est inférieure à 0,15 μm. Dans ce mode de réalisation, la composition comprend de préférence une proportion supérieure à 10% en poids, de préférence au moins 15% en poids d'agent d'émulsification par rapport au poids de polyorganosiloxane.

La taille des gouttelettes de microémulsion peut être mesurée sur une émulsion préparée préalablement à son introduction dans la composition cosmétique, par diffusion de Lumière Dynamique (DQEL), par exemple comme décrit ci-après. L'appareillage utilisé est par exemple constitué d'un laser Spectra-Physics 2020, d'un corrélateur Brookhaven 2030 et de l'informatique associée. L'échantillon étant concentré, il est dilué dans l'eau désionisée et filtré à 0,22 μm, pour être en final à 2% en poids. Le diamètre obtenu est un diamètre apparent. Les mesures sont faites à 90° et 135° d'angle. Pour les mesures de taille, outre l'analyse classique par les cumulants, trois exploitations de la fonction d'autocorrélation sont utilisées (l'échantillonnage exponentiel ou EXPSAM décrit par le Pr. Pike, la méthode « Non Negatively Constrained Least Squares » ou NNLS et la méthode CONTIN décrite par le Pr. Provencher), qui donnent chacune une répartition de taille pondérée par l'intensité diffusée, et non pas par la masse ou le nombre. Il est tenu compte de l'indice de réfraction et de la viscosité de l'eau.

Selon un mode avantageux, la microémulsion est transparente. La microémulsion peut par exemple présenter une transmittance d'au moins 90%, de préférence d'au moins 95%, à une longueur d'onde de 600 nm, mesurée par exemple à l'aide d'un spectromètre Lambda 40 UV-Vis, à une concentration à 0.5% en poids dans l'eau. Dans ce cadre, la composition cosmétique peut avantageusement être transparente. Elle peut par exemple présenter une transmittance d'au moins 90%, de préférence d'au moins 95%, à une longueur d'onde de 600 nm, mesurée par exemple à l'aide d'un spectromètre Lambda 40 UV-Vis.

Selon un autre mode particulier de réalisation, l'émulsion est une émulsion dont la taille moyenne des gouttelettes est supérieure ou égale à 0,15 μm, par exemple supérieure à 0,5 μm, ou à 1 μm, ou à 2 μm, ou à 10 μm, ou à 20 μm, et de préférence inférieure à 100 μm. La taille des gouttelettes peut être mesurée sur une émulsion préparée

préalablement à son introduction dans la composition cosmétique, ou directement sur la composition cosmétique diluée dans de l'eau, par microscopie optique et/ou granulométrie laser (Horiba LA-910 laser scattering analyzer). Dans ce mode de réalisation, la composition comprend de préférence une proportion inférieure à 10% en poids d'agent d'émulsification, par rapport au poids de polyorganosiloxane.

Des agents d'émulsification utiles pour la réalisation d'émulsion de polyorganosiloxanes sont notamment les tensioactifs non ioniques, de préférence polyalcoxylés, par exemple choisis parmi, les alcools gras alcoxylés, les triglycérides alcoxylés, les acides gras alcoxylés, les esters de sorbitan alcoxylés, les amines grasses alcoxylées, les di (phényl-1 éthyl) phénols alcoxylés, les tri(phényl-1 éthyl) phénols alcoxylés, et les alkyls phénols alcoxylés, où le nombre de motifs alcoxy, plus particulièrement oxyéthyléne et/ou oxypropyléne, est tel que la valeur de HLB est supérieure ou égale à 10.

Parmi les dérivés de silicones solubles dans l'eau de la composition, on peut citer entre autres, les diméthicones copolyols (Mirasil DMCO commercialisée par la société Rhodia Chimie).

Pour ce qui a trait aux silicones se présentant sous forme de dispersions insolubles dans l'eau de la composition, on peut utiliser de manière convenable, des organopolysiloxanes non hydrosolubles et non volatils parmi lesquels on peut citer les huiles, gommes ou résines polyalkylsiloxanes, polyarylsiloxanes, polyalkylarylsiloxanes, ou leurs dérivés fonctionnalisés non hydrosolubles, ou leurs mélanges, non volatils.

Lesdits organopolysiloxanes sont considérés comme non hydrosolubles et non volatils, lorsque leur solubilité dans l'eau est inférieure à 50 g/litre et leur viscosité intrinsèque d'au moins 3000 mPa.s, à 25°C.

A titre d'exemples d'organopolysiloxanes ou silicones non hydrosolubles et non volatils, on peut citer des gommes silicones comme par exemple la gomme diphényl diméthicone commercialisée par la société Rhodia Chimie, et de préférence les polydiméthylsiloxanes présentant une viscosité au moins égale à $6.10^5$ mPa.s, à 25°C, et de façon encore plus préférentielle, ceux d'une viscosité supérieure à $2.\ 10^6$ mPa.s, à 25°C, tels que la Mirasil DM 500000®commercialisée par la société Rhodia Chimie.

Selon l'invention, l'organopolysiloxane ou silicone non hydrosoluble et non volatil se trouve sous une forme dispersée au sein de la composition cosmétique le renfermant.

Celui-ci se présente sous forme de particules ou de gouttelettes dont la taille peut être choisie en fonction de la nature de la composition cosmétique ou de la performance recherchée pour ladite composition. D'une manière générale, cette taille peut varier de 0,01 à 70 microns.

D'une manière préférentielle, cette taille est de l'ordre de 0,1 à 50 microns, tout particulièrement de l'ordre de 1 à 30 microns.

Pour faciliter leur mise en oeuvre, ces organopolysiloxanes peuvent être préalablement dispersés ou solubilisés dans des dérivés silicones de faible viscosité, volatils ou non, puis émulsionnés dans la composition cosmétique.

Parmi ces silicones de faible viscosité, on peut citer les silicones volatils cycliques et les polydiméthyl siloxanes de faible masse.

On pourra également utiliser des dérivés de silicones fonctionnalisés comme les dérivés aminés directement sous forme d'émulsions ou à partir d'une micro-émulsion préformée. Il peut s'agir de composés connus sous le terme de silicones aminées ou de silicones hydroxylées. On cite la Mirasil ADM-E (Amodiméthicone) commercialisée par la société Rhodia, et le dimethiconol.

A titre de polyorganosiloxanes pouvant être utilisés, on cite notamment:

- les polyorganosiloxanes comprenant des unités $-Si(CH_2)_2O-$ et des unités
- $SiY(CH_2)O-$ où Y est un groupe $-(CH_2)_3-NH(CH_2)_2-NH_2$ ou $-(CH_2)_3-\ NH_2$
- les polyorganosiloxanes comprenant des unités $-Si(CH_2)_2O-$ et des unités terminales - $HO-Si(CH_2)_2O-$ et/ou des unités non terminales $-Si(CH_2)(OH)O-$
- les polyorganosiloxanes comprenant des unités $-Si(CH_2)_2O-$ et des unités
- $SiY(CH_2)O-$ où Y est $-L^X-Z^x-$Palc où $L^X$ est un groupe de liaison divalent, de préférence un groupe alkyle, $Z^X$ est une liaison covalente ou un groupe rotule divalent comprenant un hétéroatome, Palc est un groupe de formule $[OE]_s-[OP]_t-X'$, dans laquelle OE est un groupe de formule $-CH_2-CH_2-O-$, OP est un groupe de formule $-CH_2-CHCH_3-O-$ ou-$CHCH_3-CH_2-O-$, X' est un atome d'hydrogène ou un groupe de hydrocarboné, s est un nombre moyen supérieur à 1, et t est un nombre moyen supérieur ou égal à 0,
- les polyorganosiloxanes dont la chaîne comprend au moins un bloc comprenant des unités de formule des unités $-Si(CH_2)_2O-$ et au moins un bloc $-[OE]_s-[OP]_t-$,
- les polyorganosiloxanes comprenant des unités $-Si(CH_2)_2O-$ et/ou des unités
- $Si(CH_2)RO-$ et/ou $-SiR_2O-$ et/ou $R-Si(CH_2)_2O-$ et/ou $H_3C-SiR_2O-$ et/ou $R-SiR_2O-$ où R, identique ou différent est un groupe alkyle différent d'un groupe méthyle, un groupe aryle, un groupe alkyle, un groupe alkyaryl, ou un groupe aralkyl.

- Autres composés

Il est de même envisageable d'utiliser des huiles pouvant exercer des fonctions conditionnantes, protectrices, ou émollientes. De telles huiles sont généralement choisies parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin...) ou les huiles d'origine animale (suif, huiles de poisson...), des dérivés de ces huiles comme les huiles hydrogénées, les dérivés de la lanoline, le petrolatum, les huiles minérales ou les huiles paraffiniques, le perhydrosqualane, le squalène, les diols comme le 1-2-dodécanediol, l'alcool cétylique, l'alcool stéarylique, l'alcool oléique, les esters gras comme le palmitate d'isopropyl, le cocoate d'éthyl-2-hexyl, le myristyl myristate, les esters de l'acide lactique, l'acide stéarique, l'acide béhénique, l'acide isostéarique.

On peut aussi incorporer dans la composition cosmétique, sous forme de dispersions ou de solutions, des agents bactéricides ou fongicides afin d'améliorer la désinfection de la peau, comme par exemple le triclosan ; des agents anti-pelliculaires, comme notamment le zinc pyrithione ou l'octopyrox ; des agents insecticides comme les pyréthroides naturels ou de synthèse.

Les compositions cosmétiques peuvent également contenir des agents pour la protection de la peau et/ou des cheveux contre les agressions du soleil et des rayons UV. Ainsi, les compositions peuvent comprendre des filtres solaires qui sont des composés chimiques absorbant fortement le rayonnement UV, comme les composés autorisés dans la directive européenne N° 76/768/CEE, ses annexes et les modifications ultérieures de cette directive.

Dans le cas où les divers éléments constitutifs de la composition cosmétique présentent une solubilité trop faible dans la composition ou lorsqu'ils se présentent sous forme solide à température ambiante, lesdits éléments constitutifs peuvent avantageusement être solubilisés dans un véhicule organique, comme des huiles minérales ou naturelles, des dérivés de silicones; des cires, ou bien encore encapsulés dans des matrices comme des polymères de type latex.

Les compositions cosmétiques faisant l'objet de invention peuvent également contenir des résines fixatives.

Ces résines fixatives, lorsqu'elles sont présentes, le sont généralement à des concentrations comprises entre 0,01 et 10%, préférentiellement entre 0,5 et 5%.

Les résines fixatives entrant dans les compositions cosmétiques sont plus particulièrement choisies parmi les résines suivantes :

- les copolymères acrylate de méthyle / acrylamide, copolymères polyvinylméthyléther / anhydride maléique, copolymères acétate de vinyle / acide crotonique, copolymères octylacrylamide / acrylate de méthyle / butylaminoéthylméthacrylate, polyvinylpyrrolidones, copolymères polyvinylpyrrolidone / méthacrylate de méthyle, copolymères polyvinylpyrrolidone / acétate de vinyle, alcools polyvinyliques, copolymères alcool polyvinylique / acide crotonique, copolymères alcool polyvinylique / anhydride maléique, hydroxypropyl celluloses, hydroxypropyl guars, polystyrène sulfonates de sodium, terpolymères polyvinylpyrrolidone / éthyl méthacrylate/ acide méthacrylique, monométhyl éthers de poly(méthylvinyl éther / acide maléique), polyvinylacétates greffés sur des troncs polyoxyéthylènes (EP-A-219 048),
- les copolyesters dérivés d'acide, anhydride ou d'un diester téréphtalique et/ou isophtalique et/ou sulfoisophtalique et d'un diol, tels que :
    - les copolymères polyesters à base de motifs ethylène téréphtalate et/ou propylène téréphtalate et polyoxyéthylène téréphtalate, (US-A-3 959 230, US-A-3 893 929, US-A-4 116 896, US-A-4 702 857, US-A-4 770 666) ;
    - les oligomères polyesters sulfonés obtenus par sulfonation d'un oligomère dérivé de l'alcool allylique éthoxylé, du diméthyltéréphtalate et du 1,2 propylène diol (US-A-4 968 451) ;
    - les copolymères polyesters dérivés de diméthyltéréphtalate, d'acide isophtalique, de sulfoisophtalate de diméthyl et d'éthylène glycol (EP-A-540374)
    - les copolymères comprenant des unités polyesters dérivés de diméthyltéréphtalate, d'acide isophtalique, de sulfoisophtalate de diméthyl et d'éthylène glycol et d'unités polyorganosiloxanes (FR-A-2 728 915).
    - les oligomères polyesters sulfonés obtenus par condensation de l'acide isophtalique, du sulfosuccinate de diméthyle et de diéthylène glycol (FR-A-2 236 926)
    - les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896) ;
    - les polyesters-polyuréthanes obtenus par réaction d'un polyester obtenu à partir d'acide adipique et/ou d'acide téréphtalique et/ou d'acide sulfoisophtalique et d'un diol, sur un prépolymère à groupements isocyanates terminaux obtenus à partir d'un polyoxyéthylène glycol et d'un diisocyanate (FR-A-2 334 698) ;

- les monoamines ou polyamines éthoxylées, les polymères d'amines éthoxylées (US-A-4 597 898, EP-A-11 984).

De manière préférentielle, les résines fixatives sont choisies parmi les polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / palyisophtalate sulfonate de sodium, et leurs mélanges.

Ces résines fixatives sont préférentiellement dispersées ou solubilisées dans le véhicule choisi.

Les compositions cosmétiques faisant l'objet de l'invention peuvent également contenir des dérivés polymériques exerçant une fonction protectrice.

Ces dérivés polymériques peuvent être présents en quantités de l'ordre de 0,01-10%, de préférence environ 0,1-5%, et tout particulièrement de l'ordre de 0,2-3% en poids.

Ces agents peuvent notamment être choisis parmi :

. les dérivés cellulosiques non ioniques tels que les hydroxyéthers de cellulose, la méthylcellulose, l'éthylcellulose, l'hydroxypropyl méthylcellulose, l'hydroxybutyl méthylcellulose ;

. les polyvinylesters greffés sur des troncs polyalkylénés tels que les polyvinylacétates greffés sur des troncs polyoxyéthylènes (EP-A-219 048) ;

. les alcools polyvinyliques.

Les compositions cosmétiques faisant l'objet de l'invention peuvent aussi comprendre des agents plastifiants.

Lesdits agents, s'ils sont présents, peuvent représenter entre 0,1 à 20% de la formulation de préférence de 1 à 15%.

Parmi les agents plastifiants particulièrement utiles, on peut citer les adipates, les phtalates, les isophtalates, les azélates, les stéarates, les silicones copolyols, les glycols, l'huile de ricin, ou leurs mélanges.

On peut aussi avantageusement ajouter à ces compositions des agents séquestrants des métaux, plus particulièrement ceux séquestrant le calcium comme les ions citrates.

On peut également incorporer aux compositions cosmétiques faisant l'objet de l'invention des agents humectants, parmi lesquels figurent, entre autres, le glycérol, le sorbitol, l'urée, le collagène, la gélatine, l'aloe vera, l'acide hyaluronique ou des solvants volatils hydrosolubles comme l'éthanol ou le propylène glycol dont les teneurs peuvent atteindre jusqu'à 60% en poids de la composition.

Pour diminuer encore l'irritation ou l'agression du cuir chevelu, on peut aussi ajouter des polymères hydrosolubles ou hydrodispersables comme le collagène ou certains dérivés non allergisants de protéines animales ou végétales (hydrolysats de protéines de blé par exemple), des hydrocolloïdes naturels (gomme de guar, de caroube, de tara, ...) ou issus de procédés de fermentation et les dérivés de ces polycarbohydrates comme les celluloses modifiées non ioniques comme par exemple l'hydroxyéthylcellulose, ou anionique comme la carboxyméthylcellulose ; les dérivés du guar ou de la caroube comme leurs dérivés non-ioniques (par exemple hydroxypropylguar) ou les dérivés anioniques (carboxyméthylguar et carboxyméthylhydroxypropylguar).

A ces composés, on peut ajouter en association, des poudres ou des particules minérales comme du carbonate de calcium, du bicarbonate de sodium, du dihydrogénophosphate de calcium, des oxydes minéraux sous forme de poudre ou sous forme colloïdale (particules de taille inférieure ou de l'ordre de un micromètre, parfois de quelque dizaines de nanomètres) comme du dioxyde de titane, de la silice, des sels d'aluminium utilisés généralement comme anti-transpirants, du kaolin, du talc, des argiles et leurs dérivés, etc..

Des agents conservateurs comme les méthyl, éthyl, propyl et butyl esters de l'acide p-hydroxybenzoïque, le benzoate de sodium, le GERMABEN® ou tout agent chimique évitant la prolifération des bactéries ou des moisissures et utilisé traditionnellement des les compositions cosmétiques, peuvent aussi être introduits dans les compositions aqueuses cosmétiques selon l'invention, généralement à hauteur de 0,01 à 3 % en poids.

La quantité de ces produits est habituellement ajustée pour éviter toute prolifération de bactéries, moisissures ou levures dans les compositions cosmétiques.

Alternativement à ces agents chimiques, on peut parfois utiliser des agents modifiant l'activité de l'eau et augmentant fortement la pression osmotique comme les carbohydrates ou des sels.

Pour protéger la peau et/ou les cheveux des agressions du soleil et des rayons UV, on peut ajouter aux compositions des filtres solaires organiques ou minéraux, par exemple des particules minérales comme l'oxyde de zinc, le dioxyde de titane ou les oxydes de cérium sous forme de poudre ou de particules colloïdales, seuls ou en mélange. Ces poudres peuvent éventuellement être traitées en surface pour augmenter l'efficacité de leur action anti-UV ou pour faciliter leur incorporation dans les formulations cosmétiques ou pour inhiber la photoréactivité de surface. Les filtres solaires organiques peuvent notamment être introduit dans le polyorganosiloxanes, s'il est présent dans la composition.

A ces ingrédients on peut ajouter, si nécessaire, et dans le but d'augmenter le confort lors de l'utilisation de la composition par le consommateur, un ou des parfums, des agents colorants parmi lesquels on peut citer les produits

décrits dans l'annexe IV ("List of colouring agents allowed for use in cosmetic products") de la directive européenne n° 76/768/CEE du 27 juillet 1976 dite directive cosmétique, et/ou des agents opacifiants comme des pigments.

Bien que cela ne soit pas obligatoire, la composition peut aussi contenir des polymères viscosants ou gélifiants de façon à ajuster la texture de la composition, comme les polyacrylates réticulés (Carbopol commercialisés par Goodrich), déjà mentionnés ci-dessus, les dérivés non cationiques de la cellulose comme l'hydroxypropylcellulose, la carboxyméthylcellulose, les guars et leurs dérivés non ioniques, la gomme xanthane et ses dérivés, utilisés seuls ou en association, ou les mêmes composés, généralement sous la forme de polymères hydrosolubles modifiés par des groupements hydrophobes liés de manière covalente au squelette polymère comme décrit dans le brevet WO 92/16187 et/ou de l'eau pour amener le total des constituants de la formulation à 100 %.

Les compositions cosmétiques faisant l'objet de l'invention peuvent également contenir des agents dispersants polymériques en quantité de l'ordre de 0,1-7% en poids, pour contrôler la dureté en calcium et magnésium, agents tels que :

. les sels hydrosolubles d'acides polycarboxyliques de masse moléculaire en poids de l'ordre de 2000 à 100000 g/mol, obtenus par polymérisation ou copolymérisation d'acides carboxyliques éthyléniquement insaturés tels que l'acide acrylique, l'acide ou l'anhydride maléique, l'acide fumarique, l'acide itaconique, l'acide aconitique, l'acide mésaconique, l'acide citraconique, l'acide méthylènemalonique, et tout particulièrement les polyacrylates de masse moléculaire en poids de l'ordre de 2 000 à 10 000 g/mol (US-A-3 308 067), les copolymères d'acide acrylique et d'anhydride maléique de masse moléculaire en poids de l'ordre de 5000 à 75 000 g/mol (EP-A-66 915) ;
. les polyéthylèneglycols de masse moléculaire en poids de l'ordre de 1000 à 50000 g/mol.

D'autres détails ou avantages de l'invention apparaîtront plus clairement au vu des exemples qui suivent, sans caractère limitatif.

## Exemples

On réalise des compositions comprenant des ingrédients choisis parmi les suivants:

| Ingrédient | Type | Composé |
|---|---|---|
| SLES | Tensioactif anionique | laurylethersulfate de sodium (2EO), EMPICOL ESB/3M commercialisé par Hunstman |
| SLS | Tensioactif anionique | Laurylsulfate de sodium |
| ALES | Tensioactif anionique | laurylethersulfate d'ammonium (2EO), RHODAPEX-EA-2 commercialisé par Rhodia |
| ALS | Tensioactif anionique | laurylsulfate d'ammonium, RHODAPON L-22 commercialisé par Rhodia |
| CAPB | Tensioactif amphotère | Cocoamidopropylbétaine, MIRATAINE BET-C-30 commercialisé par Rhodia |
| Sel | | Chlorure de Sodium ou Chlorure d'ammonium |
| Polymère A | | Copolymère comprenant 33% en nombre d'unités dérivées de DIQUAT et 67% en nombre d'unités dérivant d'acide acrylique, de masse moléculaire d'environ 1000 000. |
| Polymère B | | Copolymère comprenant 50% en nombre d'unités dérivées de DIQUAT et 50% en nombre d'unités dérivant d'acide acrylique, de masse moléculaire d'environ 1000 000. |
| Polymère C | Polymère cationique | Cellulose cationique: JR 400 commercialisée par Amerchol - INCI PQ10 |
| Polymère D | Polymère cationique | Guar cationique: Jaguar C13S: commercialisé par Rhodia |
| Polymère E | Polymère cationique | Jaguar C162: commercialisé par Rhodia |
| Silicone 1 | Agent conditionneur | Mirasil DME-2 commercialisée par Rhodia: émulsion de dimethicone (PDMS) de viscosité d'environ 500000 cP, de taille des gouttelettes d'environ 2 μm, stabilisée par du succinoglycane |

(suite)

| Ingrédient | Type | Composé |
|---|---|---|
| Silicone 2 | Agent conditionneur | Mirasil DME-30 commercialisée par Rhodia: émulsion de dimethicone (PDMS) de viscosité d'environ 500000 cP, de taille des gouttelettes d'environ 2 μm, stabilisée par du succinoglycane |

Mode opératoire

1. Mélanger l'eau et le polymère
2. Ajouter le CAPB
3. Ajouter le tensioactif anionique, puis éventuellement l'émulsion de silicone
4. Ajuster le pH à 6-6,5 par ajout de d'hydroxyde de sodium ou d'acide citrique
5. Ajouter le sel

On réalise les compositions suivantes, dont la quantité en poids de chaque ingrédient est donnée ci-dessous (la lettre C indique des exemples comparatifs):

| Exemple | 1 | 2 | 3 | 4C | 5 | 6 | 7 | 8C | 9 | 10 | 11C |
|---|---|---|---|---|---|---|---|---|---|---|---|
| SLES (%) | 14 | 14 | 14 | 14 | / | / | / | / | 8 | 8 | 8 |
| SLS (%) | / | / | / | / | / | / | / | / | / | / | / |
| ALES (%) | / | / | / | / | 7 | 7 | 7 | 7 | / | / | / |
| ALS (%) | / | / | / | / | 7 | 7 | 7 | 7 | / | / | / |
| CAPB (%) | 2 | 2 | 2 | 2 | 2 | 2 | 2 | 2 | 2 | 2 | 2 |
| $NH_4Cl$ (%) | / | / | / | / | 0,25 | / | / | 0,25 | / | / | / |
| NaCl (%) | 1,5 | 1,5 | 1,5 | 1,5 | / | / | / | / | 3 | 3 | 3 |
| Polymère A (%) | 0,3 | 1 | / | / | 0,3 | 1 | / | / | 0,5 | 0,5 | / |
| Polymère B (%) | / | / | 1 | / | / | / | 1 | / | / | / | / |
| Eau | Jusqu'à 100% | | | | | | | | | | |

| | 12 | 13 | 14 | 15C | 16C | 17C | 18C | 19C | 20C |
|---|---|---|---|---|---|---|---|---|---|
| SLES (%) | 14 | 14 | 14 | 14 | 14 | 14 | 14 | 14 | 14 |
| CAPB (%) | 2 | 2 | 2 | 2 | 2 | 2 | 2 | 2 | 2 |
| NaCl (%) | 1,5 | 1,5 | 1,5 | 1,5 | 1,5 | 1,5 | 1,5 | 1,5 | 1,5 |
| Polymère A (%) | 0,1 | 0,2 | 0,3 | / | / | / | | | |
| Polymère C (%) | | | | 0,1 | 0,2 | 0,3 | | | |
| Polymère E (%) | | | | | | | 0,1 | 0,2 | 0,3 |
| Silicone 1 (% matière sèche) | 1 | 1 | 1 | 1 | 1 | 1 | 1 | 1 | 1 |
| Silicone 2 (% matière sèche) | / | / | / | / | / | / | / | / | / |
| Eau | Jusqu'à 100% | | | | | | | | |

On mesure la transmittance (transparence) des compositions à 600 nm à l'aide d'un spectrophotomètre (type Jasco 7800), dans des cellules de 10*10 mm, ou dans des cellules de 10*100 mm.

On mesure la viscosité Brookfield des compositions, à l'aide d'un viscosimètre Brookfield DV-I, à 22°C, 10 tours par minute, spindle 4 pour des viscosités inférieures à 11000 mPa.s, spindle 5 pour des viscosités de 11000 à 30000 mPa.s, spindle 6 au delà.

| Exemple | 30C | 31C | 32C | 24C | 25C | 26C | 27 | 28 | 29 |
|---|---|---|---|---|---|---|---|---|---|
| Transmittance 10*10 mm | 99 | 99 | 99 | 98 | 95 | 92 | 98 | 95 | 94 |
| Transmittance 10*100 mm | 90 | 90 | 88 | 76 | 55 | 41 | 75 | 65 | 54 |

## Revendications

**1.** Utilisation cosmétique d'une composition cosmétique comprenant:

- un vecteur cosmétiquement acceptable, de préférence de l'eau,
- un copolymère ampholyte comprenant des unités cationiques (c) et des unités (a) anioniques ou susceptibles d'être anioniques, et
- éventuellement un tensioactif,

**caractérisée en ce que:**

- le copolymère comprend:

  - 0,1 à 50% en nombre d'unités (c), dérivant de la polymérisation d'au moins un composé monomère (c) de formule générale I :

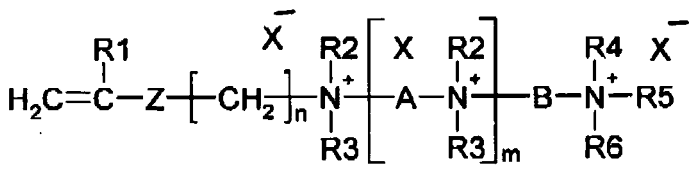

dans laquelle:

- $R_1$ est un atome d'hydrogène, un groupe méthyle ou éthyle ;
- $R_2$, $R_3$, $R_4$, $R_5$, $R_6$, identiques ou différents, sont des groupes alkyle, hydroxyalkyle ou aminoalkyle, linéaires ou ramifiés, en $C_1$-$C_6$, de préférence en $C_1$-$C_4$,
- m est un nombre entier de 0 à 10, de préférence de 0 à 2 ;
- n est un nombre entier de 1 à 6, de préférence 2 à 4 ;
- Z représente un groupe -C(O)O-, -C(O)NH- ou un atome d'oxygène ;
- A représente un groupe $(CH_2)_p$, p étant un nombre entier de 1 à 6, de préférence de 2 à 4;
- B représente une chaîne polyméthylène linéaire ou ramifiée en $C_2$-$C_{12}$, avantageusement $C_3$-$C_6$, éventuellement interrompue par un ou plusieurs hétéroatomes ou hétérogroupes, notamment O ou NH, et éventuellement substituée par un ou plusieurs groupes hydroxyles ou amino, de préférence hydroxyles ;
- $X^-$, identiques ou différents, représentent des contre-ions ;

  - des unités (a) dérivant de la polymérisation d'au moins un monomère (a) hydrophile portant une fonction à caractère acide copolymérisable avec (a), anionique ou susceptible d'être anionique,
  - éventuellement des unités (n) dérivant d'au moins un monomère (n) à insaturation éthylénique, de charge neutre, copolymérisable avec (c) et (a), de préférence un composé monomère hydrophile à insaturation éthylénique de charge neutre portant un ou plusieurs groupes hydrophiles, copolymérisable avec (c) et (a),
  - la quantité d'unités (a) et éventuellement (n) étant de 50 à 99,9 % en nombre,
  - le pH de la composition est supérieur ou égal à 5,5, de préférence compris entre 5,5 et 7,5,

- la proportion en poids de tensioactif dans la composition est comprise entre 0 et 30% de préférence entre, 5 et 30% en poids, le tensioactif comprenant un tensioactif anionique et éventuellement un tensioactif amphotère,
- la proportion en poids du copolymère dans la composition est comprise entre 0,01 et 5%, de préférence entre 0,05 et 1,5%, de préférence de 0,1 à 0,3%.

**2.** Utilisation selon la revendication 1, **caractérisée en ce que** la composition est un shampoing comprenant entre 10 et 20% de tensioactif, ou d'un gel-douche comprenant entre 5 et 15% de tensioactif, ou d'un après shampoing comprenant moins de 5% de tensioactif.

**3.** Utilisation selon l'une des revendications précédentes, **caractérisée en ce que :**

- les unités (c ) dérivent de monomère (c ) de formule suivante:

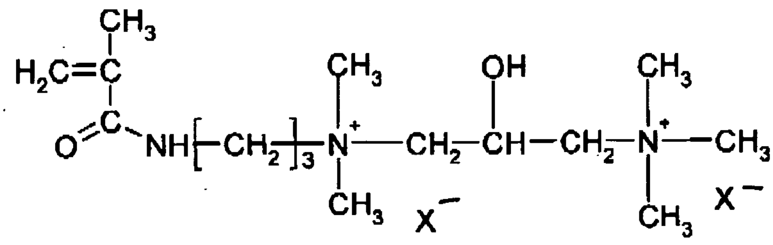

$X^-$ représentant l'ion chlorure ou méthyl sulfate,
- les unités (a) dérivent d'acide acrylique,
- le polymère ne comprend pas d'unités (n),
- le rapport en nombre entre les unités (a) et les unités (c) est de 50/50 à 90/10.

**4.** Utilisation selon l'une des revendications précédentes **caractérisée en ce que** le tensioactif anionique est choisi parmi les alkylesters sulfonates, les alkylbenzènes sulfonates, les alkylsulfates, les alkylethersulfates, les alkamides sulfates, les alkylamidoethersulfates, les acides gras, les N-acyl Nalkyltaurates, les alkyliséthionates, les alkylsuccinamates, les alkylsulfosuccinates, les monoesters ou diesters de sulfosuccinates, les N-acyl sarcosinates, les polyethoxycarboxylates, les mono et di esters phosphates, et leurs sels, seuls ou en mélanges.

**5.** Utilisation selon l'une des revendications précédentes **caractérisée en ce que** la composition comprend un tensioactif amphotère choisi parmi les bétaïnes, les sulfo-bétaïnes, les alkylamphoacétates, les alkylamphopropionates, les alkyl amphohydroxypropyl sultaines.

**6.** Utilisation selon l'une des revendications précédentes, **caractérisée en ce que** la composition comprend 12 à 16% en poids d'alkylethersulfate de sodium ou d'un mélange d'alkylethersulfate de sodium et d'alkylsulfate de sodium, 1 à 3 % d'un tensioactif amphotère, et 0.5 à 2 % d'un sel.

**7.** Utilisation selon l'une des revendications précédentes, **caractérisée en ce que** la composition comprend 12 à 16% en poids d'alkylethersulfate d'ammonium ou d'un mélange d'alkylethersuffate d'ammonium et d'alkylsulfate d'ammonium, 1 à 3 % d'un tensioactif amphotère, et 0 à 2 % d'un sel.

**8.** Utilisation selon l'une des revendications précédentes, **caractérisée en ce que** la composition comprend 6 à 10% en poids d'alkylethersulfate de sodium ou d'un mélange d'alkylethersulfate de sodium et d'alkylsulfate de sodium, 1 à 3 % d'un tensioactif amphotère, et 2 à 4 % d'un sel.

**9.** Utilisation selon l'une des revendications précédentes, **caractérisée en ce que** la composition comprend un polyorganosiloxane.

**10.** Utilisation selon la revendication 9, **caractérisée en ce que** le polyorganosiloxane est un polyorganosiloxane non volatil et non-hydrosoluble.

**11.** Utilisation selon la revendication 9, **caractérisé en ce que** le polyorganosiloxane présente une viscosité comprise entre 1000 et 2000000 mPa.s, de préférence entre 5000 et 500000 mPa.s.

**12.** Utilisation selon l'une des revendications 9 à 11, **caractérisée en ce que** le polyorganosiloxane est un polydiméthylorganosiloxane, ou un polyorganosiloxane présentant des groupes amines, ammonium quaternaire, hydroxyle, ou polyoxyalkylène, ou plusieurs de ces groupes.

**13.** Utilisation selon l'une des revendications 9 à 12, **caractérisée en ce que** le silicone est sous forme d'une émulsion de gouttelettes.

**14.** Utilisation selon la revendication 13, **caractérisé en ce que** l'émulsion est une émulsion dont la taille moyenne des gouttelettes est supérieure ou égale à 2 μm, et/ou dont la taille moyenne des gouttelettes est supérieure ou comprise entre 0,15 μm et 2 μm, ou dont la taille moyenne des gouttelettes est inférieure ou égale à 0,15 μm.

**15.** Utilisation selon la revendication 14, **caractérisée en ce que** l'émulsion est une émulsion dont la taille moyenne

des gouttelettes est supérieure ou égale à 0,15 µm, obtenue éventuellement à l'aide d'un agent d'émulsification de préférence en proportion inférieure à 10%, en poids par rapport à la quantité de polyorganosiloxane, ou l'émulsion est une microémulsion dont la taille moyenne des gouttelettes est inférieure à 0,15 µm, obtenue à l'aide d'un agent d'émulsification de préférence en proportion supérieure à 10% en poids par rapport à la quantité de polyorganosiloxane,

**16.** Utilisation selon l'une des revendications précédentes **caractérisée en ce que** la composition comprend un agent de stabilisation et/ou d'aide au conditionnement autre que le polyorganosiloxane, de préférence un polyacrylate réticulé ou un solide insoluble formant un réseau dans la composition.

**17.** Utilisation selon l'une des revendications précédentes, **caractérisée en ce que** le copolymère est utilisé dans la composition à titre d'agent à déposer sur des cheveux ou sur la peau, ou à titre d'agent d'aide au dépôt d'agents conditionneurs, sur les cheveux ou sur la peau.

**18.** Utilisation selon la revendication 17, **caractérisée en ce que** le copolymère est utilisé dans la composition à titre d'agent transparent.

**19.** Utilisation selon l'une des revendications 16 ou 17, **caractérisée en ce que** le copolymère est utilisé dans la composition à titre d'agent d'aide au dépôt d'agents conditionneurs, et **en ce que** l'agent conditionneur est un polyorganosiloxane selon l'une des revendications 9 à 15.

**20.** Utilisation selon l'une des revendications précédentes, **caractérisée en ce que** la composition est une composition destinée à être rincée, de préférence un shampoing, un après-shampoing ou un gel-douche.

## Claims

**1.** Cosmetic use of a cosmetic composition comprising:

- a cosmetically acceptable vector, preferably water,
- an ampholytic copolymer comprising cationic units (c) and anionic or potentially anionic units (a), and
- optionally a surfactant

**characterized in that**:

- the copolymer comprises:

  - 0.1% to 50% by number of units (c) derived from the polymerization of at least one monomeric compound (c) of general formula I:

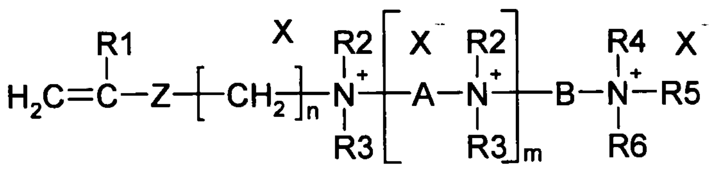

in which:

- $R_1$ is a hydrogen atom or a methyl or ethyl group;
- $R_2$, $R_3$, $R_4$, $R_5$ and $R_6$, which may be identical or different, are linear or branched $C_1$-$C_6$ and preferably $C_1$-$C_4$ alkyl, hydroxyalkyl or aminoalkyl groups,
- m is an integer from 0 to 10 and preferably from 0 to 2;
- n is an integer from 1 to 6 and preferably from 2 to 4;
- Z represents a -C(O)O- or -C(O)NH- group or an oxygen atom;
- A represents a group $(CH_2)_p$, p being an integer from 1 to 6 and preferably from 2 to 4;
- B represents a linear or branched $C_2$-$C_{12}$ and advantageously $C_3$-$C_6$ polymethylene chain, optionally interrupted with one or more hetero atoms or hetero groups, especially O or NH, and

optionally substituted with one or more hydroxyl or amino groups, preferably hydroxyl groups;
- $X^-$, which may be identical or different, represent counterions;

- units (a) derived from the polymerization of at least one hydrophilic monomer (a) bearing a function of acidic nature that is copolymerizable with (a), which is anionic or potentially anionic,
- optionally units (n) derived from at least one ethylenically unsaturated monomer (n) of neutral charge, which is copolymerizable with (c) and (a), preferably an ethylenically unsaturated hydrophilic monomer compound of neutral charge bearing one or more hydrophilic groups, which is copolymerizable with (c) and (a),
- the amount of units (a) and optionally (n) being from 50% to 99.9% by number,
- the pH of the composition is greater than or equal to 5.5 and preferably between 5.5 and 7.5,

- the weight proportion of surfactant in the composition is between 0 and 30% and preferably between 5% and 30% by weight, the surfactant comprising an anionic surfactant and optionally an amphoteric surfactant,
- the weight proportion of the copolymer in the composition is between 0.01% and 5%, preferably between 0.05% and 1.5% and preferentially from 0.1% to 0.3%.

**2.** The use as claimed in claim 1, **characterized in that** it is a shampoo comprising between 10% and 20% of surfactant, or a shower gel comprising between 5% and 15% of surfactant, or a conditioner comprising less than 5% of surfactant.

**3.** The use as claimed in either of the preceding claims, **characterized in that**:

- the units (c ) are derived from a monomer (c) of the following formula:

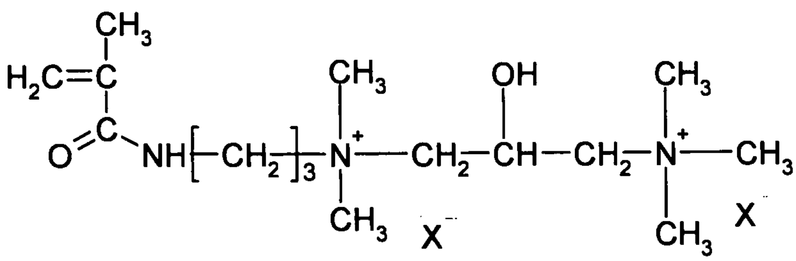

$X^-$ representing a chloride or methyl sulfate ion,
- the units (a) are derived from acrylic acid,
- the polymer does not comprise any units (n),
- the numerical ratio between the units (a) and the units (c) is from 50/50 to 90/10.

**4.** The use as claimed in any one of the preceding claims, **characterized in that** the anionic surfactant is chosen from alkyl ester sulfonates, alkylbenzenesulfonates, alkyl sulfates, alkyl ether sulfates, alkylamide sulfates, alkylamidoether sulfates, fatty acids, N-acyl-N-alkyltaurates, alkylisethionates, alkylsuccinamates, alkylsulfosuccinates, sulfosuccinate monoesters or diesters, N-acyl sarcosinates, polyethoxycarboxylates and phosphate monoesters and diesters, and salts thereof, alone or as mixtures.

**5.** The use as claimed in one of the preceding claims, **characterized in that** it comprises an amphoteric surfactant chosen from betaines, sulfobetaines, alkylamphoacetates, alkylamphopropionates and alkylamphohydroxypropyl sultaines.

**6.** The use as claimed in one of the preceding claims, **characterized in that** it comprises 12% to 16% by weight of sodium alkyl ether sulfate or of a mixture of sodium alkyl ether sulfate and of sodium alkyl sulfate, 1% to 3% of an amphoteric surfactant and 0.5% to 2% of a salt.

**7.** The use as claimed in one of the preceding claims, **characterized in that** it comprises 12% to 16% by weight of ammonium alkyl ether sulfate or of a mixture of ammonium alkyl ether sulfate and of ammonium alkyl sulfate, 1% to 3% of an amphoteric surfactant and 0 to 2% of a salt.

**8.** The use as claimed in one of the preceding claims, **characterized in that** it comprises 6% to 10% by weight of

sodium alkyl ether sulfate or of a mixture of sodium alkyl ether sulfate and of sodium alkyl sulfate, 1% to 3% of an amphoteric surfactant and 2% to 4% of a salt.

**9.** The use as claimed in one of the preceding claims, **characterized in that** it comprises a polyorganosiloxane.

**10.** The use as claimed in claim 9, **characterized in that** the polyorganosiloxane is a water-insoluble nonvolatile polyorganosiloxane.

**11.** The use as claimed in claim 9, **characterized in that** the polyorganosiloxane has a viscosity of between 1000 and 2 000 000 mPa.s and preferably between 5000 and 500 000 mPa.s.

**12.** The use as claimed in one of claims 9 to 11, **characterized in that** the polyorganosiloxane is a polydimethylorganosiloxane, or a polyorganosiloxane containing amine, quaternary ammonium, hydroxyl or polyoxyalkylene groups, or several of these groups.

**13.** The use as claimed in one of claims 9 to 12, **characterized in that** the silicone is in the form of an emulsion of droplets.

**14.** The use as claimed in claim 13, **characterized in that** the emulsion is an emulsion whose mean droplet size is greater than or equal to 2 μm, and/or whose mean droplet size is greater than or between 0.15 μm and 2 μm, or whose mean droplet size is less than or equal to 0.15 μm.

**15.** The use as claimed in claim 14, **characterized in that** the emulsion is an emulsion whose mean droplet size is greater than or equal to 0.15 μm, optionally obtained using an emulsifier preferably in a proportion of less than 10% by weight relative to the amount of polyorganosiloxane, or

- the emulsion is a microemulsion whose mean droplet size is less than 0.15 μm, obtained using an emulsifier preferably in a proportion of greater than 10% by weight relative to the amount of polyorganosiloxane.

**16.** The use as claimed in one of the preceding claims, **characterized in that** it comprises a stabilizer and/or conditioning aid other than the polyorganosiloxane, preferably a crosslinked polyacrylate or an insoluble solid forming a network in the composition.

**17.** The use as claimed in one of the preceding claims, **characterized in that** the copolymer is used in the composition, as an agent to be deposited onto the hair or the skin, or as an agent for aiding the deposition of conditioning agents on the hair or the skin.

**18.** The use as claimed in claim 17, as a transparent agent.

**19.** The use as claimed in either of claims 16 and 17, as an agent for aiding the deposition of conditioning agents, **characterized in that** the conditioning agent is a polyorganosiloxane as claimed in one of claims 9 to 15.

**20.** The use as claimed in one of the preceding claims, **characterized in that** the composition is a composition intended to be rinsed out, preferably a shampoo, a hair conditioner or a shower gel.

## Patentansprüche

**1.** Kosmetische Verwendung einer kosmetischen Zusammensetzung, umfassend:

- einen kosmetisch annehmbaren Träger, vorzugsweise Wasser,
- ein Ampholyt-Copolymer, welches Einheiten (c), welche kationisch sind, und Einheiten (a), welche anionisch sind oder anionisch sein können, umfasst, und
- gegebenenfalls einen grenzflächenaktiven Stoff,

**dadurch gekennzeichnet, dass**:

- das Copolymer umfasst:

  - 0,1 bis 50%, bezogen auf die Anzahl, von Einheiten (c), die sich ableiten aus der Polymerisation von

mindestens einer Monomer-Verbindung (c) der allgemeinen Formel 1:

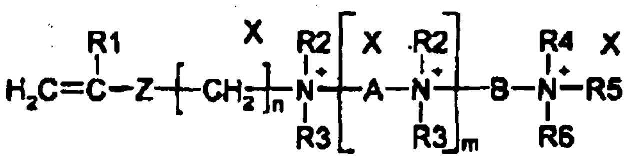

,

in welcher:

- $R_1$ ein Wasserstoffatom, eine Methyl- oder Ethylgruppe ist;
- $R_2$, $R_3$, $R_4$, $R_5$, $R_6$, die gleich oder voneinander verschieden sind, lineare oder verzweigte $C_1$-$C_8$-, vorzugsweise $C_1$-$C_4$-Alkyl-, -Hydroxyalkyl- oder
- Aminoalkylgruppen sind;
- m eine ganze Zahl von 0 bis 10, vorzugsweise von 0 bis 2 ist;
- n eine ganze Zahl von 1 bis 6, vorzugsweise 2 bis 4 ist;
- Z für eine Gruppe -C(O)O-, -C(0)NH- oder ein Sauerstoffatom steht;
- A für eine Gruppe $(CH_2)_p$ steht, wobei p eine ganze Zahl von 1 bis 6, vorzugsweise von 2 bis 4 ist;
- B für eine lineare oder verzweigte $C_2$-$C_{12}$-, vorteilhafterweise $C_3$-$C_8$-Polymethylenkette, die gegebenenfalls durch ein(e) oder mehrere Heteroatome oder Heterogruppen, insbesondere O oder NH, unterbrochen ist und gegebenenfalls durch eine oder mehrere Hydroxyl- oder Aminogruppen, vorzugsweise Hydroxylgruppen substituiert ist, steht;
- $X^-$, die gleich oder voneinander verschieden sind, für Gegenionen stehen;

- Einheiten (a), die sich aus der Polymerisation von mindestens einem hydrophilen Monomer (a), welches eine Funktion mit saurem Charakter, welche mit (a) copolymerisierbar ist, welche anionisch ist oder anionisch sein kann, trägt, ableiten,
- gegebenenfalls Einheiten (n), welche sich ableiten von mindestens einem Monomer (n) mit ethylenischer Ungesättigtheit und von neutraler Ladung, welches mit (c) und (a) copolymerisierbar ist, vorzugsweise einer hydrophilen Monomer-Verbindung mit ethylenischer Ungesättigtheit und von neutraler Ladung, welche eine oder mehrere hydrophile Gruppen trägt, welche mit (c) und (a) copolymerisierbar ist,
- wobei die Menge von Einheiten (a) und gegebenenfalls (n) 50 bis 99,9%, bezogen auf die Anzahl, beträgt,

- der pH der Zusammensetzung höher als oder gleich 5,5 ist, vorzugsweise zwischen 5,5 und 7,5 liegt,
- der Gewichtsanteil von grenzflächenaktivem Stoff in der Zusammensetzung zwischen 0 und 30 Gew.-%, vorzugsweise zwischen 5 und 30 Gew.-% liegt, wobei der grenzflächenaktive Stoff einen anionischen grenzflächenaktiven Stoff und gegebenenfalls einen amphoteren grenzflächenaktiven Stoff umfasst,
- der Gewichtsanteil des Copolymers in der Zusammensetzung zwischen 0,01 und 5%, vorzugsweise zwischen 0,05 und 1,5% liegt, vorzugsweise 0,1 bis 0,3% beträgt.

2. Verwendung nach Anspruch 1, **dadurch gekennzeichnet, dass** die Zusammensetzung ein Shampoo, welches zwischen 10 und 20% grenzflächenaktiven Stoff umfasst, oder ein Duschgel, welches zwischen 5 und 15% grenzflächenaktiven Stoff umfasst, oder ein nach dem Shampoo anzuwendendes Produkt, welches weniger als 5% grenzflächenaktiven Stoff umfasst, ist.

3. Verwendung nach einem der vorangegangenen Ansprüche, **dadurch gekennzeichnet, dass**:

- die Einheiten (c) sich von Monomer (c) der folgenden Formel:

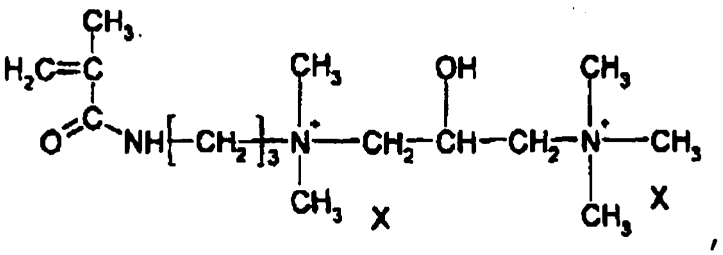

wobei X- für das Chloridion oder Methylsulfat steht, ableiten,

- die Einheiten (a) sich von Acrylsäure ableiten,
- das Polymer keine Einheiten (n) umfasst,
- das auf die Anzahl bezogene Verhältnis zwischen den Einheiten (a) und den Einheiten (c) 50/50 bis 90/10 beträgt.

4. Verwendung nach einem der vorangegangenen Ansprüche, **dadurch gekennzeichnet, dass** der anionische grenzflächenaktive Stoff unter den Alkylestersulfonaten, den Alkylbenzolsulfonaten, den Alkylsulfaten, den Alkylethersulfaten, den Alkamidsulfaten, den Alkylamidoethersulfaten, den Fettsäuren, den N-Acyl-N-alkyltauraten, den Alkylisethionaten, den Alkylsuccinamaten, den Alkylsulfosuccinaten, den Monoestern oder Diestern von Sulfosuccinaten, den N-Acylsarcosinaten, den Polyethoxycarboxylaten, den Mono- und Diesterphosphaten und deren Salzen, allein oder in Mischungen, ausgewählt ist.

5. Verwendung nach einem der vorangegangenen Ansprüche, **dadurch gekennzeichnet, dass** die Zusammensetzung einen amphoteren grenzflächenaktiven Stoff, der unter den Betainen, den Sulfobetainen, den Alkylamphoacetaten, den Alkylamphopropionaten, den Alkylamphohydroxypropylsultainen ausgewählt ist, umfasst.

6. Verwendung nach einem der vorangegangenen Ansprüche, **dadurch gekennzeichnet, dass** die Zusammensetzung 12 bis 16 Gew.-% Natriumalkylethersulfat oder einer Mischung von Natriumalkylethersulfat und Natriumalkylsulfat, 1 bis 3% eines amphoteren grenzflächenaktiven Stoffs und 0,5 bis 2% eines Salzes umfasst.

7. Verwendung nach einem der vorangegangenen Ansprüche, **dadurch gekennzeichnet, dass** die Zusammensetzung 12 bis 16 Gew.-% Ammoniumalkylethersulfat oder einer Mischung von Ammoniumalkylethersulfat und Ammoniumalkylsulfat, 1 bis 3% eines amphoteren grenzflächenaktiven Stoffs und 0 bis 2% eines Salzes umfasst.

8. Verwendung nach einem der vorangegangenen Ansprüche, **dadurch gekennzeichnet, dass** die Zusammensetzung 6 bis 10 Gew.-% Natriumalkylethersulfat oder einer Mischung von Natriumalkylethersulfat und von Natriumalkylsulfat, 1 bis 3% eines amphoteren grenzflächenaktiven Stoffs und 2 bis 4% eines Salzes umfasst.

9. Verwendung nach einem der vorangegangenen Ansprüche, **dadurch gekennzeichnet, dass** die Zusammensetzung ein Polyorganosiloxan umfasst.

10. Verwendung nach Anspruch 9, **dadurch gekennzeichnet, dass** das Polyorganosiloxan ein nicht-flüchtiges und nicht-wasserlösliches Polyorganosiloxan ist.

11. Verwendung nach Anspruch 9, **dadurch gekennzeichnet, dass** das Polyorganosiloxan eine Viskosität zwischen 1000 und 2000000 mPa.s, vorzugsweise zwischen 5000 und 500000 mPa.s aufweist.

12. Verwendung nach einem der Ansprüche 9 bis 11, **dadurch gekennzeichnet, dass** das Polyorganosiloxan ein Polydimethylorganosiloxan oder ein Polyorganosiloxan, welches Amin-, quartäre Ammonium-, Hydroxyl- oder Polyoxyalkylengruppen oder mehrere von diesen Gruppen aufweist, ist.

13. Verwendung nach einem der Ansprüche 9 bis 12, **dadurch gekennzeichnet, dass** das Silicon in Form einer Emulsion von Tröpfchen vorliegt.

14. Verwendung nach Anspruch 13, **dadurch gekennzeichnet, dass** die Emulsion eine Emulsion ist, bei der die mittlere

Größe der Tröpfchen größer als oder gleich 2 µm ist und/oder bei der die mittlere Größe der Tröpfchen größer als oder zwischen 0,15 µm und 2 µm ist oder bei der die mittlere Größe der Tröpfchen kleiner als oder gleich 0,15 µm ist.

**15.** Verwendung nach Anspruch 14, **dadurch gekennzeichnet, dass** die Emulsion eine Emulsion ist, bei der die mittlere Größe der Tröpfchen größer als oder gleich 0,15 µm ist, welche gegebenenfalls mit Hilfe eines Emulgiermittels vorzugsweise in einem Anteil unter 10 Gew.-% bezogen auf die Menge von Polyorganosiloxan erhalten wird, oder

- die Emulsion eine Mikroemulsion ist, bei der die mittlere Größe der Tröpfchen unter 0,15 µm liegt, welche mit Hilfe eines Emulgiermittels vorzugsweise in einem Anteil über 10 Gew.-% bezogen auf die Menge von Polyorganosiloxan erhalten wird.

**16.** Verwendung nach einem der vorangegangenen Ansprüche, **dadurch gekennzeichnet, dass** die Zusammensetzung ein Stabilisierungs- und/oder Konditionierhilfsmittel außer dem Polyorganosiloxan, vorzugsweise ein vernetztes Polyacrylat oder einen unlöslichen Feststoff, welcher in der Zusammensetzung ein Netzwerk bildet, umfasst.

**17.** Verwendung nach einem der vorangegangenen Ansprüche, **dadurch gekennzeichnet, dass** das Copolymer in der Zusammensetzung als Mittel, das sich auf Haaren oder auf der Haut abscheiden soll, oder als Mittel, das die Abscheidung von Konditionierungsmitteln auf dem Haar oder auf der Haut unterstützt, eingesetzt wird.

**18.** Verwendung nach Anspruch 17, **dadurch gekennzeichnet, dass** das Copolymer in der Zusammensetzung als transparentes Mittel eingesetzt wird.

**19.** Verwendung nach einem der Ansprüche 16 oder 17, **dadurch gekennzeichnet, dass** das Copolymer in der Zusammensetzung als ein Mittel, das die Abscheidung von Konditionierungsmitteln unterstützt, eingesetzt wird und dass das Konditionierungsmittel ein Polyorganosiloxan gemäß einem der Ansprüche 9 bis 15 ist.

**20.** Verwendung nach einem der vorangegangenen Ansprüche, **dadurch gekennzeichnet, dass** die Zusammensetzung eine Zusammensetzung, die dazu bestimmt ist, abgespült zu werden, vorzugsweise ein Shampoo, ein nach dem Shampoo anzuwendendes Produkt oder ein Duschgel ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 269243 A
- EP 266111 A
- EP 521748 A
- EP 522755 A
- EP 522756 A
- EP 1115370 A
- US 6569261 B1
- US 4387017 A
- EP 156646 A
- US 4565647 A
- EP 219048 A
- US 3959230 A
- US 3893929 A
- US 4116896 A
- US 4702857 A
- US 4770666 A
- US 4968451 A
- EP 540374 A
- FR 2728915 A
- FR 2236926 A
- US 4711730 A
- US 4721580 A
- US 4877896 A
- FR 2334698 A
- US 4597898 A
- EP 11984 A
- WO 9216187 A
- US 3308067 A
- EP 66915 A


### Littérature non-brevet citée dans la description


- **G. Robinson ; S.B. Ross Murphy ; E.R. Morris.** Viscosity-Molecular weight relationship, intrinsic chain flexibility and dynamic solution properties of guar galactomannan. *Carbohydrate Research,* 1982, vol. 107, 17-32